# EUROPEAN PATENT SPECIFICATION

(11) **EP 1 653 915 B1**
(45) Date of publication and mention of the grant of the patent: **26.05.2010**
(21) Application number: 04781103.9
(22) Date of filing: 13.08.2004
(51) Int. Cl.: C07K 16/00, C12Q 1/00

(54) **PRKCS AS MODIFIERS OF THE BETA CATENIN PATHWAY AND METHODS OF USE**
PRKCS ALS MODIFIKATOREN DES BETA CATENIN PFADS UND ANWENDUNGSVERFAHREN
PRKC UTILISES EN TANT QUE MODIFICATEURS DE LA VOIE BETA CATENINE ET METHODES D'UTILISATION ASSOCIEES

(30) Priority: 14.08.2003 US 495172 P
(43) Date of publication of application: 10.05.2006
(73) Proprietor: Exelixis, Inc., South San Francisco, CA 94083-0511 (US)
(72) Inventor: FRANCIS-LANG, Helen, San Francisco, California 94109 (US); WINTER, Christopher G., Needham, MA 02492 (US); VENTURA, Richard Benn Abegania, Daly City, California 94014 (US); HEUER, Timothy S., El Granada, CA 94018 (US); ADAMKEWICZ, Joanne I., Novato, CA 94947 (US)
(74) Representative: Main, Malcolm Charles
(86) International application number: PCT/US2004/026361
(87) International publication number: WO 2005/016282

(56) References cited:
- WO-A-02/058450
- WO-A-03/023048
- WO-A-03/052068
- US-A1- 2002 015 943
- WILLIAMS CAROL L ET AL: "Reduced expression of Wnt-1 and E-cadherin, and diminished beta-catenin stability in MCF-7 breast cancer cells that overexpress protein kinase C-alpha" INTERNATIONAL JOURNAL OF ONCOLOGY, vol. 19, no. 6, December 2001 (2001-12), pages 1227-1233, XP002394272 ISSN: 1019-6439
- REN JIAN ET AL: "Protein kinase C delta regulates function of the DF3/MUC1 carcinoma antigen in beta-catenin signaling" JOURNAL OF BIOLOGICAL CHEMISTRY, vol. 277, no. 20, 17 May 2002 (2002-05-17), pages 17616-17622, XP002394273 ISSN: 0021-9258
- CHEN R H ET AL: 'Wnt signaling to b-catenin involves two interactive components.' J BIOL CHEM. vol. 275, 2000, pages 17894 - 17899, XP002998509
- MONGA S P S ET AL: 'Beta-catenin antisense studies in embryonic liver cultures: role in proliferation, apoptosis, and lineage specification.' GASTROENTEROLOGY. vol. 124, 2003, pages 202 - 216, XP002998510

### SEQUENCE LISTING

<110> EXELIXIS, INC.
<120> PRKCS AS MODIFIERS OF THE BETA CATENIN PATHWAY AND METHODS OF USE
<130> EX04-056C-PC
<150> US 60/495,172
   <151> 2003-08-14
<160> 12
<170> PatentIn version 3.2
<210> 1
   <211> 2261
   <212> DNA
   <213> Homo sapiens
<400> 1
   ccgcggttcc ggctgctccg gcgaggcgac ccttgggtcg gcgctgcggg cgaggtgggc      60
   aggtaggtgg gcggacggcc gcggttctcc ggcaagcgca ggcggcggag tcccccacgg     120
   cgcccgaagc gccccccgca cccccggcct ccagcgttga ggcgggggag tgaggagatg      180
   ccgacccaga gggacagcag caccatgtcc cacacggtcg caggcggcgg cagcggggac     240
   cattcccacc aggtccgggt gaaagcctac taccgcgggg atatcatgat aacacatttt     300
   gaaccttcca tctcctttga gggcctttgc aatgaggttc gagacatgtg ttcttttgac     360
   aacgaacagc tcttcaccat gaaatggata gatgaggaag gagacccgtg tacagtatca     420
   tctcagttgg agttagaaga agcctttaga ctttatgagc taaacaagga ttctgaactc     480
   ttgattcatg tgttcccttg tgtaccagaa cgtcctggga tgccttgtcc aggagaagat     540
   aaatccatct accgtagagg tgcacgccgc tggagaaagc tttattgtgc caatggccac     600
   actttccaag ccaagcgttt caacaggcgt gctcactgtg ccatctgcac agaccgaata     660
   tggggacttg gacgccaagg atataagtgc atcaactgca aactcttggt tcataagaag     720
   tgccataaac tcgtcacaat tgaatgtggg cggcattctt tgccacagga accagtgatg     780
   cccatggatc agtcatccat gcattctgac catgcacaga cagtaattcc atataatcct     840
   tcaagtcatg agagtttgga tcaagttggt gaagaaaaag aggcaatgaa caccagggaa     900
   agtggcaaag cttcatccag tctaggtctt caggattttg atttgctccg ggtaatagga     960
   agaggaagtt atgccaaagt actgttggtt cgattaaaaa aaacagatcg tatttatgca    1020
   atgaaagttg tgaaaaaaga gcttgttaat gatgatgagg atattgattg ggtacagaca    1080
   gagaagcatg tgtttgagca ggcatccaat catcctttcc ttgttgggct gcattcttgc 1140
   tttcagacag aaagcagatt gttctttgtt atagagtatg taaatggagg agacctaatg 1200
   tttcatatgc agcgacaaag aaaacttcct gaagaacatg ccagatttta ctctgcagaa 1260
   atcagtctag cattaaatta tcttcatgag cgagggataa tttatagaga tttgaaactg 1320
   gacaatgtat tactggactc tgaaggccac attaaactca ctgactacgg catgtgtaag 1380
   gaaggattac ggccaggaga tacaaccagc actttctgtg gtactcctaa ttacattgct 1440
   cctgaaattt taagaggaga agattatggt ttcagtgttg actggtgggc tcttggagtg 1500
   ctcatgtttg agatgatggc aggaaggtct ccatttgata ttgttgggag ctccgataac 1560
   cctgaccaga acacagagga ttatctcttc caagttattt tggaaaaaca aattcgcata 1620
   ccacgttctc tgtctgtaaa agctgcaagt gttctgaaga gttttcttaa taaggaccct 1680
   aaggaacgat tgggttgtca tcctcaaaca ggatttgctg atattcaggg acacccgttc 1740
   ttccgaaatg ttgattggga tatgatggag caaaaacagg tggtacctcc ctttaaacca 1800
   aatatttctg gggaatttgg tttggacaac tttgattctc agtttactaa tgaacctgtc 1860
   cagctcactc cagatgacga tgacattgtg aggaagattg atcagtctga atttgaaggt 1920
   tttgagtata tcaatcctct tttgatgtct gcagaagaat gtgtctgatc ctcatttttc 1980
   aaccatgtat tctactcatg ttgccattta atgcatggat aaacttgctg caagcctgga 2040
   tacaattaac cattttatat ttgccaccta caaaaaaaca cccaatatct tctcttgtag 2100
   actatatgaa tcaattatta catctgtttt actatgaaaa aaaaattaat actactagct 2160
   tccagacaat catgtcaaaa tttagttgaa ctggtttttc agtttttaaa aggcctacag 2220
   atgagtaatg aagttacctt ttttgtttaa aaaaaaaaaa g 2261
<210> 2
   <211> 2325
   <212> DNA
   <213> Homo sapiens
<400> 2
   agcggttttg ggcccgggcg gctgtagagg cggcggcgcc tacgggcagt gggaggagcc 60
   gcgcggttcc ggctgctccg gcgaggcgac ccttgggtcg gcgctgcggg cgaggtgggc 120
   aggtaggtgg gcggacggcc gcggttctcc ggcaagcgca ggcggcggag tcccccacgg 180
   cgcccgaagc gcccccccgc acccccggcc tccagcgttg aggcggggga gtgaggagat 240
   gccgacccag agggacagca gcaccatgtc ccacacggtc gcaggcggcg gcagcgggga 300
   ccattcccac caggtccggg tgaaagccta ctaccgcggg gatatcatga taacacattt   360
   tgaaccttcc atctcctttg agggcctttg caatgaggtt cgagacatgt gttcttttga   420
   caacgaacag ctcttcacca tgaaatggat agatgaggaa ggagacccgt gtacagtatc   480
   atctcagttg gagttagaag aagcctttag actttatgag ctaaacaagg attctgaact   540
   cttgattcat gtgttccctt gtgtaccaga acgtcctggg atgccttgtc caggagaaga   600
   taaatccatc taccgtagag gtgcacgccg ctggagaaag ctttattgtg ccaatggcca   660
   cactttccaa gccaagcgtt tcaacaggcg tgctcactgt gccatctgca cagaccgaat   720
   atggggactt ggacgccaag gatataagtg catcaactgc aaactcttgg ttcataagaa   780
   gtgccataaa ctcgtcacaa ttgaatgtgg gcggcattct ttgccacagg aaccagtgat   840
   gcccatggat cagtcatcca tgcattctga ccatgcacag acagtaattc catataatcc   900
   ttcaagtcat gagagtttgg atcaagttgg tgaagaaaaa gaggcaatga acaccaggga   960
   aagtggcaaa gcttcatcca gtctaggtct tcaggatttt gatttgctcc gggtaatagg  1020
   aagaggaagt tatgccaaag tactgttggt tcgattaaaa aaaacagatc gtatttatgc  1080
   aatgaaagtt gtgaaaaaag agcttgttaa tgatgatgag gatattgatt gggtacagac  1140
   agagaagcat gtgtttgagc aggcatccaa tcatcctttc cttgttgggc tgcattcttg  1200
   ctttcagaca gaaagcagat tgttctttgt tatagagtat gtaaatggag gagacctaat  1260
   gtttcatatg cagcgacaaa gaaaacttcc tgaagaacat gccagatttt actctgcaga  1320
   aatcagtcta gcattaaatt atcttcatga gcgagggata atttatagag atttgaaact  1380
   ggacaatgta ttactggact ctgaaggcca cattaaactc actgactacg gcatgtgtaa  1440
   ggaaggatta cggccaggag atacaaccag cactttctgt ggtactccta attacattgc  1500
   tcctgaaatt ttaagaggag aagattatgg tttcagtgtt gactggtggg ctcttggagt  1560
   gctcatgttt gagatgatgg caggaaggtc tccatttgat attgttggga gctccgataa  1620
   ccctgaccag aacacagagg attatctctt ccaagttatt ttggaaaaac aaattcgcat  1680
   accacgttct atgtctgtaa aagctgcaag tgttctgaag agttttctta ataaggaccc  1740
   taaggaacga ttgggttgtc ttcctcaaac aggatttgct gatattcagg gacacccgtt  1800
   cttccgaaat gttgattggg atatgatgga gcaaaaacag gtggtacctc cctttaaacc  1860
   aaatatttct ggggaatttg gtttggacaa ctttgattct cagtttacta atgaacgtgt  1920
   ccagctcact ccagatgacg atgacattgt gaggaagatt gatcagtctg aatttgaagg  1980
   ttttgagtat atcaatcctc ttttgatgtc tgcagaagaa tgtgtctgat cctcattttt  2040
   caaccatgta ttctactcat gttgccattt aatgcatgga taaacttgct gcaagcctgg   2100
   atacaattaa ccattttata tttgccacct acaaaaaaac acccaatatc ttctcttgta   2160
   gactatatga atcaattatt acatctgttt tactatgaaa aaaaaattaa tactactagc   2220
   ttccagacaa tcatgtcaaa atttagttga actggttttt cagtttttaa aaggcctaca   2280
   gatgagtaat gaagttatct tttttgttta aaaaaaaaaa aaaaa                   2325
<210> 3
   <211> 2261
   <212> DNA
   <213> Homo sapiens
<400> 3
   ccgcggttcc ggctgctccg gcgaggcgac ccttgggtcg gcgctgcggg cgaggtgggc     60
   aggtaggtgg gcggacggcc gcggttctcc ggcaagcgca ggcggcggag tcccccacgg    120
   cgcccgaagc gccccccgca cccccggcct ccagcgttga ggcgggggag tgaggagatg    180
   ccgacccaga gggacagcag caccatgtcc cacacggtcg caggcggcgg cagcggggac    240
   cattcccacc aggtccgggt gaaagcctac taccgcgggg atatcatgat aacacatttt    300
   gaaccttcca tctcctttga gggcctttgc aatgaggttc gagacatgtg ttcttttgac    360
   aacgaacagc tcttcaccat gaaatggata gatgaggaag gagacccgtg tacagtatca    420
   tctcagttgg agttagaaga agcctttaga ctttatgagc taaacaagga ttctgaactc    480
   ttgattcatg tgttcccttg tgtaccagaa cgtcctggga tgccttgtcc aggagaagat    540
   aaatccatct accgtagagg tgcacgccgc tggagaaagc tttattgtgc caatggccac    600
   actttccaag ccaagcgttt caacaggcgt gctcactgtg ccatctgcac agaccgaata    660
   tggggacttg gacgccaagg atataagtgc atcaactgca aactcttggt tcataagaag    720
   tgccataaac tcgtcacaat tgaatgtggg cggcattctt tgccacagga accagtgatg    780
   cccatggatc agtcatccat gcattctgac catgcacaga cagtaattcc atataatcct    840
   tcaagtcatg agagtttgga tcaagttggt gaagaaaaag aggcaatgaa caccagggaa    900
   agtggcaaag cttcatccag tctaggtctt caggattttg atttgctccg ggtaatagga    960
   agaggaagtt atgccaaagt actgttggtt cgattaaaaa aaacagatcg tatttatgca   1020
   atgaaagttg tgaaaaaaga gcttgttaat gatgatgagg atattgattg ggtacagaca   1080
   gagaagcatg tgtttgagca ggcatccaat catcctttcc ttgttgggct gcattcttgc   1140
   tttcagacag aaagcagatt gttctttgtt atagagtatg taaatggagg agacctaatg   1200
   tttcatatgc agcgacaaag aaaacttcct gaagaacatg ccagatttta ctctgcagaa   1260
   atcagtctag cattaaatta tcttcatgag cgagggataa tttatagaga tttgaaactg   1320
   gacaatgtat tactggactc tgaaggccac attaaactca ctgactacgg catgtgtaag   1380
   gaaggattac ggccaggaga tacaaccagc actttctgtg gtactcctaa ttacattgct   1440
   cctgaaattt taagaggaga agattatggt ttcagtgttg actggtgggc tcttggagtg   1500
   ctcatgtttg agatgatggc aggaaggtct ccatttgata ttgttgggag ctccgataac   1560
   cctgaccaga acacagagga ttatctcttc caagttattt tggaaaaaca aattcgcata   1620
   ccacgttctc tgtctgtaaa agctgcaagt gttctgaaga gttttcttaa taaggaccct   1680
   aaggaacgat tgggttgtca tcctcaaaca ggatttgctg atattcaggg acacccgttc   1740
   ttccgaaatg ttgattggga tatgatggag caaaaacagg tggtacctcc ctttaaacca   1800
   aatatttctg gggaatttgg tttggacaac tttgattctc agtttactaa tgaacctgtc   1860
   cagctcactc cagatgacga tgacattgtg aggaagattg atcagtctga atttgaaggt   1920
   tttgagtata tcaatcctct tttgatgtct gcagaagaat gtgtctgatc ctcatttttc   1980
   aaccatgtat tctactcatg ttgccattta atgcatggat aaacttgctg caagcctgga   2040
   tacaattaac cattttatat ttgccaccta caaaaaaaca cccaatatct tctcttgtag   2100
   actatatgaa tcaattatta catctgtttt actatgaaaa aaaaattaat actactagct   2160
   tccagacaat catgtcaaaa tttagttgaa ctggtttttc agtttttaaa aggcctacag   2220
   atgagtaatg aagttacctt ttttgtttaa aaaaaaaaaa g                         2261
<210> 4
   <211> 2320
   <212> DNA
   <213> Homo sapiens
<400> 4
   ttttgggccc gggcggctgt agaggcggcg gcgcctacgg gcagtgggag gagccgcgcg     60
   gttccggctg ctccggcgag gcgacccttg ggtcggcgct gcgggcgagg tgggcaggta    120
   ggtgggcgga cggccgcggt tctccggcaa gcgcaggcgg cggagtcccc cacggcgccc    180
   gaagcgcccc cccgcacccc cggcctccag cgttgaggcg ggggagtgag gagatgccga    240
   cccagaggga cagcagcacc atgtcccaca cggtcgcagg cggcggcagc ggggaccatt    300
   cccaccaggt ccgggtgaaa gcctactacc gcggggatat catgataaca cattttgaac    360
   cttccatctc ctttgagggc ctttgcaatg aggttcgaga catgtgttct tttgacaacg    420
   aacagctctt caccatgaaa tggatagatg aggaaggaga cccgtgtaca gtatcatctc 480
   agttggagtt agaagaagcc tttagacttt atgagctaaa caaggattct gaactcttga 540
   ttcatgtgtt cccttgtgta ccagaacgtc ctgggatgcc ttgtccagga gaagataaat 600
   ccatctaccg tagaggtgca cgccgctgga gaaagcttta ttgtgccaat ggccacactt 660
   tccaagccaa gcgtttcaac aggcgtgctc actgtgccat ctgcacagac cgaatatggg 720
   gacttggacg ccaaggatat aagtgcatca actgcaaact cttggttcat aagaagtgcc 780
   ataaactcgt cacaattgaa tgtgggcggc attctttgcc acaggaacca gtgatgccca 840
   tggatcagtc atccatgcat tctgaccatg cacagacagt aattccatat aatccttcaa 900
   gtcatgagag tttggatcaa gttggtgaag aaaaagaggc aatgaacacc agggaaagtg 960
   gcaaagcttc atccagtcta ggtcttcagg attttgattt gctccgggta ataggaagag 1020
   gaagttatgc caaagtactg ttggttcgat taaaaaaaac agatcgtatt tatgcaatga 1080
   aagttgtgaa aaaagagctt gttaatgatg atgaggatat tgattgggta cagacagaga 1140
   agcatgtgtt tgagcaggca tccaatcatc ctttccttgt tgggctgcat tcttgctttc 1200
   agacagaaag cagattgttc tttgttatag agtatgtaaa tggaggagac ctaatgtttc 1260
   atatgcagcg acaaagaaaa cttcctgaag aacatgccag attttactct gcagaaatca 1320
   gtctagcatt aaattatctt catgagcgag ggataattta tagagatttg aaactggaca 1380
   atgtattact ggactctgaa ggccacatta aactcactga ctacggcatg tgtaaggaag 1440
   gattacggcc aggagataca accagcactt tctgtggtac tcctaattac attgctcctg 1500
   aaattttaag aggagaagat tatggtttca gtgttgactg gtgggctctt ggagtgctca 1560
   tgtttgagat gatggcagga aggtctccat ttgatattgt tgggagctcc gataaccctg 1620
   accagaacac agaggattat ctcttccaag ttattttgga aaaacaaatt cgcataccac 1680
   gttctatgtc tgtaaaagct gcaagtgttc tgaagagttt tcttaataag gaccctaagg 1740
   aacgattggg ttgtcttcct caaacaggat ttgctgatat tcagggacac ccgttcttcc 1800
   gaaatgttga ttgggatatg atggagcaaa aacaggtggt acctcccttt aaaccaaata 1860
   tttctgggga atttggtttg gacaactttg attctcagtt tactaatgaa cgtgtccagc 1920
   tcactccaga tgacgatgac attgtgagga agattgatca gtctgaattt gaaggttttg 1980
   agtatatcaa tcctcttttg atgtctgcag aagaatgtgt ctgatcctca tttttcaacc 2040
   atgtattcta ctcatgttgc catttaatgc atggataaac ttgctgcaag cctggataca 2100
   attaaccatt ttatatttgc cacctacaaa aaaacaccca atatcttctc ttgtagacta 2160
   tatgaatcaa ttattacatc tgttttacta tgaaaaaaaa attaatacta ctagcttcca 2220
   gacaatcatg tcaaaattta gttgaactgg tttttcagtt tttaaaaggc ctacagatga 2280
   gtaatgaagt tatctttttt gtttaaaaaa aaaaaaaaaa 2320
<210> 5
   <211> 2320
   <212> DNA
   <213> Homo sapiens
<400> 5
   ttttgggccc gggcggctgt agaggcggcg gcgcctacgg gcagtgggag gagccgcgcg 60
   gttccggctg ctccggcgag gcgacccttg ggtcggcgct gcgggcgagg tgggcaggta 120
   ggtgggcgga cggccgcggt tctccggcaa gcgcaggcgg cggagtcccc cacggcgccc 180
   gaagcgcccc cccgcacccc cggcctccag cgttgaggcg ggggagtgag gagatgccga 240
   cccagaggga cagcagcacc atgtcccaca cggtcgcagg cggcggcagc ggggaccatt 300
   cccaccaggt ccgggtgaaa gcctactacc gcggggatat catgataaca cattttgaac 360
   cttccatctc ctttgagggc ctttgcaatg aggttcgaga catgtgttct tttgacaacg 420
   aacagctctt caccatgaaa tggatagatg aggaaggaga cccgtgtaca gtatcatctc 480
   agttggagtt agaagaagcc tttagacttt atgagctaaa caaggattct gaactcttga 540
   ttcatgtgtt cccttgtgta ccagaacgtc ctgggatgcc ttgtccagga gaagataaat 600
   ccatctaccg tagaggtgca cgccgctgga gaaagcttta ttgtgccaat ggccacactt 660
   tccaagccaa gcgtttcaac aggcgtgctc actgtgccat ctgcacagac cgaatatggg 720
   gacttggacg ccaaggatat aagtgcatca actgcaaact cttggttcat aagaagtgcc 780
   ataaactcgt cacaattgaa tgtgggcggc attctttgcc acaggaacca gtgatgccca 840
   tggatcagtc atccatgcat tctgaccatg cacagacagt aattccatat aatccttcaa 900
   gtcatgagag tttggatcaa gttggtgaag aaaaagaggc aatgaacacc agggaaagtg 960
   gcaaagcttc atccagtcta ggtcttcagg attttgattt gctccgggta ataggaagag 1020
   gaagttatgc caaagtactg ttggttcgat taaaaaaaac agatcgtatt tatgcaatga 1080
   aagttgtgaa aaaagagctt gttaatgatg atgaggatat tgattgggta cagacagaga 1140
   agcatgtgtt tgagcaggca tccaatcatc ctttccttgt tgggctgcat tcttgctttc 1200
   agacagaaag cagattgttc tttgttatag agtatgtaaa tggaggagac ctaatgtttc 1260
   atatgcagcg acaaagaaaa cttcctgaag aacatgccag attttactct gcagaaatca   1320
   gtctagcatt aaattatctt catgagcgag ggataattta tagagatttg aaactggaca   1380
   atgtattact ggactctgaa ggccacatta aactcactga ctacggcatg tgtaaggaag   1440
   gattacggcc aggagataca accagcactt tctgtggtac tcctaattac attgctcctg   1500
   aaattttaag aggagaagat tatggtttca gtgttgactg gtgggctctt ggagtgctca   1560
   tgtttgagat gatggcagga aggtctccat ttgatattgt tgggagctcc gataaccctg   1620
   accagaacac agaggattat ctcttccaag ttattttgga aaaacaaatt cgcataccac   1680
   gttctatgtc tgtaaaagct gcaagtgttc tgaagagttt tcttaataag gaccctaagg   1740
   aacgattggg ttgtcttcct caaacaggat ttgctgatat tcagggacac ccgttcttcc   1800
   gaaatgttga ttgggatatg atggagcaaa aacaggtggt acctcccttt aaaccaaata   1860
   tttctgggga atttggtttg gacaactttg attctcagtt tactaatgaa cgtgtccagc   1920
   tcactccaga tgacgatgac attgtgagga agattgatca gtctgaattt gaaggttttg   1980
   agtatatcaa tcctcttttg atgtctgcag aagaatgtgt ctgatcctca tttttcaacc   2040
   atgtattcta ctcatgttgc catttaatgc atggataaac ttgctgcaag cctggataca   2100
   attaaccatt ttatatttgc cacctacaaa aaaacaccca atatcttctc ttgtagacta   2160
   tatgaatcaa ttattacatc tgttttacta tgaaaaaaaa attaatacta ctagcttcca   2220
   gacaatcatg tcaaaattta gttgaactgg tttttcagtt tttaaaaggc ctacagatga   2280
   gtaatgaagt tatctttttt gtttaaaaaa aaaaaaaaaa                         2320
<210> 6
   <211> 2164
   <212> DNA
   <213> Homo sapiens
<400> 6
   atgcccagca ggaccgaccc caagatggaa gggagcggcg gccgcgtccg cctcaaggcg     60
   cattacgggg gggacatctt catcaccagc gtggacgccg ccacgacctt cgaggagctc    120
   tgtgaggaag tgagagacat gtgtcgtctg caccagcagc acccgctcac cctcaagtgg    180
   gtggacagcg aaggtgaccc ttgcacggtg tcctcccaga tggagctgga agaggctttc    240
   cgcctggccc gtcagtgcag ggatgaaggc ctcatcattc atgttttccc gagcacccct    300
   gagcagcctg gcctgccatg tccgggagaa gacaaatcta tctaccgccg gggagccaga    360
   agatggagga agctgtaccg tgccaacggc cacctcttcc aagccaagcg ctttaacagg    420
   agagcgtact gcggtcagtg cagcgagagg atatggggcc tcgcgaggca aggctacagg    480
   tgcatcaact gcaaactgct ggtccataag cgctgccacg gcctcgtccc gctgacctgc    540
   aggaagcata tggattctgt catgccttcc caagagcctc cagtagacga caagaacgag    600
   gacgccgacc ttccttccga ggagacagat ggaattgctt acatttcctc atcccggaag    660
   catgacagca ttaaagacga ctcggaggac cttaagccag ttatcgatgg gatggatgga    720
   atcaaaatct ctcaggggct tgggctgcag gactttgacc taatcagagt catcgggcgc    780
   gggagctacg ccaaggttct cctggtgcgg ttgaagaaga atgaccaaat ttacgccatg    840
   aaagtggtga agaaagagct ggtgcatgat gacgaggata ttgactgggt acagacagag    900
   aagcacgtgt ttgagcaggc atccagcaac cccttcctgg tcggattaca ctcctgcttc    960
   cagacgacaa gtcggttgtt cctggtcatt gagtacgtca acggcgggga cctgatgttc   1020
   cacatgcaga ggcagaggaa gctccctgag gagcacgcca ggttctacgc ggccgagatc   1080
   tgcatcgccc tcaacttcct gcacgagagg gggatcatct acagggacct gaagctggac   1140
   aacgtcctcc tggatgcgga cgggcacatc aagctcacag actacggcat gtgcaaggaa   1200
   ggcctgggcc ctggtgacac aacgagcact ttctgcggaa ccccgaatta catcgccccc   1260
   gaaatcctgc ggggagagga gtacgggttc agcgtggact ggtgggcgct gggagtcctc   1320
   atgtttgaga tgatggccgg gcgctccccg ttcgacatca tcaccgacaa cccggacatg   1380
   aacacagagg actacctttt ccaagtgatc ctggagaagc ccatccggat cccccggttc   1440
   ctgtccgtca aagcctccca tgttttaaaa ggatttttaa ataaggaccc caaagagagg   1500
   ctcggctgcc ggccacagac tggattttct gacatcaagt cccacgcgtt cttccgcagc   1560
   atagactggg acttgctgga gaagaagcag gcgctccctc cattccagcc acagatcaca   1620
   gacgactacg gtctggacaa ctttgacaca cagttcacca gcgagcccgt gcagctgacc   1680
   ccagacgatg aggatgccat aaagaggatc gaccagtcag agttcgaagg ctttgagtat   1740
   atcaacccat tattgctgtc caccgaggag tcggtgtgag gccgcgtgcg tctctgtcgt   1800
   ggacacgcgt gattgaccct ttaactgtat ccttaaccac cgcatatgca tgccaggctg   1860
   ggcacggctc cgagggcggc cagggacaga cgcttgcgcc gagaccgcag agggaagcgt   1920
   cagcgggcgc tgctgggagc agaacagtcc ctcacacctg gcccggcagg cagcttcgtg   1980
   ctggaggaac ttgctgctgt gcctgcgtcg cggcggatcc gcggggaccc tgccgagggg   2040
   gctgtcatgc ggtttccaag gtgcacattt tccacggaaa cagaactcga tgcactgacc   2100
   tgctccgcca ggaaagtgag cgtgtagcgt cctgaggaat aaaatgttcc gatgaaaaaa   2160
   aaaa 2164
<210> 7
   <211> 2315
   <212> DNA
   <213> Homo sapiens
<400> 7
   ggcacgaggc tgagcgctgc cttccgcgtt ccgccgcggc cccacctgga gcccccgccc 60
   cgcgccatgg ccggagctcc cggggcgcag cgctgacggc ggcgggggga gcgcgccatg 120
   cccagcagga ccggccccaa gatggaaggg agcggcggcc gcgtccgcct caaggcgcat 180
   tacggggggg acatcttcat caccagcgtg gacgccgcca cgaccttcga ggagctctgt 240
   gaggaagtga gagacatgtg tcgtctgcac cagcagcacc cgctcaccct caagtgggtg 300
   gacagcgaag gtgacccttg cacggtgtcc tcccagatgg agctggaaga ggctttccgc 360
   ctggcccgtc agtgcaggga tgaaggcctc atcattcatg ttttcccgag cacccctgag 420
   cagcctggcc tgccatgtcc gggagaagac aaatctatct accgccgggg agccagaaga 480
   tggaggaagc tgtaccgtgc caacggccac ctcttccaag ccaagcgctt taacaggaga 540
   gcgtactgcg gtcagtgcag cgagaggata tggggcctcg cgaggcaagg ctacaggtgc 600
   atcaactgca aactgctggt ccataagcgc tgccacggcc tcgtcccgct gacctgcagg 660
   aagcatatgg attctgtcat gccttcccaa gagcctccag tagacgacaa gaacgaggac 720
   gccgaccttc cttccgagga gacagatgga attgcttaca tttcctcatc ccggaagcat 780
   gacagcatta aagacgactc ggaggacctt aagccagtta tcgatgggat ggatggaatc 840
   aaaatctctc aggggcttgg gctgcaggac tttgacctaa tcagagtcat cgggcgcggg 900
   agctacgcca aggttctcct ggtgcggttg aagaagaatg accaaattta cgccatgaaa 960
   gtggtgaaga aagagctggt gcatgatgac gaggatattg actgggtaca gacagagaag 1020
   cacgtgtttg agcaggcatc cagcaacccc ttcctggtcg gattacactc ctgcttccag 1080
   acgacaagtc ggttgttcct ggtcattgag tacgtcaacg gcggggacct gatgttccac 1140
   atgcagaggc agaggaagct ccctgaggag cacgccaggt tctacgcggc cgagatctgc 1200
   atcgccctca acttcctgca cgagaggggg atcatctaca gggacctgaa gctggacaac 1260
   gtcctcctgg atgcggacgg gcacatcaag ctcacagact acggcatgtg caaggaaggc 1320
   ctgggccctg gtgacacaac gagcactttc tgcggaaccc cgaattacat cgcccccgaa 1380
   atcctgcggg gagaggagta cgggttcagc gtggactggt gggcgctggg agtcctcatg 1440
   tttgagatga tggccgggcg ctccccgttc gacatcatca ccgacaaccc ggacatgaac 1500
   acagaggact accttttcca agtgatcctg gagaagccca tccggatccc ccggttcctg   1560
   tccgtcaaag cctcccatgt tttaaaagga tttttaaata aggaccccaa agagaggctc   1620
   ggctgccggc cacagactgg attttctgac atcaagtccc acgcgttctt ccgcagcata   1680
   gactgggact tgctggagaa gaagcaggcg ctccctccat tccagccaca gatcacagac   1740
   gactacggtc tggacaactt tgacacacag ttcaccagcg agcccgtgca gctgacccca   1800
   gacgatgagg atgccataaa gaggatcgac cagtcagagt tcgaaggctt tgagtatatc   1860
   aacccattat tgctgtccac cgaggagtcg gtgtgaggcc gcgtgcgtct ctgtcgtgga 1920
   cacgcgtgat tgacccttta actgtatcct taaccaccgc atatgcatgc caggctgggc 1980
   acggctccga gggcggccag ggacagacgc ttgcgccgag accgcagagg gaagcgtcag   2040
   cgggcgctgc tgggagcaga acagtccctc acacctgggc ccgggcaggc cagcttcgtg   2100
   ctggaggaac ttgctgctgt gcctgcgtcg cggcggatcc gcggggaccc tgccgagggg   2160
   gctgtcatgc ggtttccaag gtgcacattt tccacggaaa cagaactcga tgcactgacc   2220
   tgctccgcca ggaaagtgag cgtgtagcgt cctgaggaat aaaatgttcc gatgatgtgg   2280
   aaaaaaaaaa aaaaaaaaaa aaaaaaaaaa aaaaa                              2315
<210> 8
   <211> 1779
   <212> DNA
   <213> Homo sapiens
<400> 8
   atgcccagca ggaccgaccc caagatggaa gggagcggcg gccgcgtccg cctcaaggcg     60
   cattacgggg gggacatctt catcaccagc gtggacgccg ccacgacctt cgaggagctc    120
   tgtgaggaag tgagagacat gtgtcgtctg caccagcagc acccgctcac cctcaagtgg    180
   gtggacagcg aaggtgaccc ttgcacggtg tcctcccaga tggagctgga agaggctttc    240
   cgcctggccc gtcagtgcag ggatgaaggc ctcatcattc atgttttccc gagcacccct    300
   gagcagcctg gcctgccatg tccgggagaa gacaaatcta tctaccgccg gggagccaga    360
   agatggagga agctgtaccg tgccaacggc cacctcttcc aagccaagcg ctttaacagg    420
   agagcgtact gcggtcagtg cagcgagagg atatggggcc tcgcgaggca aggctacagg    480
   tgcatcaact gcaaactgct ggtccataag cgctgccacg gcctcgtccc gctgacctgc    540
   aggaagcata tggattctgt catgccttcc caagagcctc cagtagacga caagaacgag    600
   gacgccgacc ttccttccga ggagacagat ggaattgctt acatttcctc atcccggaag 660
   catgacagca ttaaagacga ctcggaggac cttaagccag ttatcgatgg gatggatgga 720
   atcaaaatct ctcaggggct tgggctgcag gactttgacc taatcagagt catcgggcgc     780
   gggacgtacg ccaaggttct cctggtgcgg ttgaagaaga atgaccaaat ttacgccatg     840
   aaagtggtga agaaagagct ggtgcatgat gacgaggata ttgactgggt acagacagag 900
   aagcacgtgt ttgagcaggc atccagcaac cccttcctgg tcggattaca ctcctgcttc 960
   cagacgacaa gtcggttgtt cctggtcatt gagtacgtca acggcgggga cctgatgttc 1020
   cacatgcaga ggcagaggaa gctccctgag gagcacgcca ggttctacgc ggccgagatc 1080
   tgcatcgccc tcaacttcct gcacgagagg gggatcatct acagggacct gaagctggac 1140
   aacgtcctcc tggatgcgga cggacacatc aagctcacag actacggcat gtgcaaggaa 1200
   ggcctgggcc ctggtgacac aacgagcact ttctgcggaa ccccgaatta catcgccccc 1260
   gaaatcctgc ggggagagga gtacgggttc agcgtggact ggtgggcgct gggagtcctc 1320
   atgtttgaga tgatggccgg gcgctccccg ttcgacatca tcaccgacaa cccggacatg 1380
   aacacagagg actacctttt ccaagtgatc ctggagaagc ccatccggat cccccggttc 1440
   ctgtccgtca aagcctccca tgttttaaaa ggatttttaa ataaggaccc caaagagagg 1500
   ctcggctgcc ggccacagac tggattttct gacatcaagt cccacgcgtt cttccgcagc 1560
   atagactggg acttgctgga gaagaagcag gcgctccctc cattccagcc acagatcaca 1620
   gacgactacg gtctggacaa ctttgacaca cagttcacca gcgagcccgt gcagctgacc 1680
   ccagacgatg aggatgccat aaagaggatc gaccagtcag agttcgaagg ctttgagtat 1740
   atcaacccat tattgctgtc caccgaggag tcggtgtga 1779
<210> 9
   <211> 2306
   <212> DNA
   <213> Homo sapiens
<400> 9
   ctgagcgctg ccttccgcgt tccgccgcgg ccccacctgg agcccccgcc ccgcgccatg 60
   gccggagctc ccggggcgca gcgctgacgg cggcgggggg agcgcgccat gcccagcagg 120
   accggcccca agatggaagg gagcggcggc cgcgtccgcc tcaaggcgca ttacgggggg 180
   gacatcttca tcaccagcgt ggacgccgcc acgaccttcg aggagctctg tgaggaagtg 240
   agagacatgt gtcgtctgca ccagcagcac ccgctcaccc tcaagtgggt ggacagcgaa 300
   ggtgaccctt gcacggtgtc ctcccagatg gagctggaag aggctttccg cctggcccgt 360
   cagtgcaggg atgaaggcct catcattcat gttttcccga gcacccctga gcagcctggc 420
   ctgccatgtc cgggagaaga caaatctatc taccgccggg gagccagaag atggaggaag 480
   ctgtaccgtg ccaacggcca cctcttccaa gccaagcgct ttaacaggag agcgtactgc 540
   ggtcagtgca gcgagaggat atggggcctc gcgaggcaag gctacaggtg catcaactgc 600
   aaactgctgg tccataagcg ctgccacggc ctcgtcccgc tgacctgcag gaagcatatg 660
   gattctgtca tgccttccca agagcctcca gtagacgaca agaacgagga cgccgacctt 720
   ccttccgagg agacagatgg aattgcttac atttcctcat cccggaagca tgacagcatt 780
   aaagacgact cggaggacct taagccagtt atcgatggga tggatggaat caaaatctct 840
   caggggcttg ggctgcagga ctttgaccta atcagagtca tcgggcgcgg gagctacgcc 900
   aaggttctcc tggtgcggtt gaagaagaat gaccaaattt acgccatgaa agtggtgaag 960
   aaagagctgg tgcatgatga cgaggatatt gactgggtac agacagagaa gcacgtgttt 1020
   gagcaggcat ccagcaaccc cttcctggtc ggattacact cctgcttcca gacgacaagt 1080
   cggttgttcc tggtcattga gtacgtcaac ggcggggacc tgatgttcca catgcagagg 1140
   cagaggaagc tccctgagga gcacgccagg ttctacgcgg ccgagatctg catcgccctc 1200
   aacttcctgc acgagagggg gatcatctac agggacctga agctggacaa cgtcctcctg 1260
   gatgcggacg ggcacatcaa gctcacagac tacggcatgt gcaaggaagg cctgggccct 1320
   ggtgacacaa cgagcacttt ctgcggaacc ccgaattaca tcgcccccga aatcctgcgg 1380
   ggagaggagt acgggttcag cgtggactgg tgggcgctgg gagtcctcat gtttgagatg 1440
   atggccgggc gctccccgtt cgacatcatc accgacaacc cggacatgaa cacagaggac 1500
   taccttttcc aagtgatcct ggagaagccc atccggatcc cccggttcct gtccgtcaaa 1560
   gcctcccatg ttttaaaagg atttttaaat aaggacccca aagagaggct cggctgccgg 1620
   ccacagactg gattttctga catcaagtcc cacgcgttct tccgcagcat agactgggac 1680
   ttgctggaga agaagcaggc gctccctcca ttccagccac agatcacaga cgactacggt 1740
   ctggacaact ttgacacaca gttcaccagc gagcccgtgc agctgacccc agacgatgag 1800
   gatgccataa agaggatcga ccagtcagag ttcgaaggct ttgagtatat caacccatta 1860
   ttgctgtcca ccgaggagtc ggtgtgaggc cgcgtgcgtc tctgtcgtgg acacgcgtga 1920
   ttgacccttt aactgtatcc ttaaccaccg catatgcatg ccaggctggg cacggctccg 1980
   agggcggcca gggacagacg cttgcgccga gaccgcagag ggaagcgtca gcgggcgctg 2040
   ctgggagcag aacagtccct cacacctggg cccgggcagg ccagcttcgt gctggaggaa 2100
   cttgctgctg tgcctgcgtc gcggcggatc cgcggggacc ctgccgaggg ggctgtcatg 2160
   cggtttccaa ggtgcacatt ttccacggaa acagaactcg atgcactgac ctgctccgcc 2220
   aggaaagtga gcgtgtagcg tcctgaggaa taaaatgttc cgatgatgtg gaaaaaaaaa 2280
   aaaaaaaaaa aaaaaaaaaa aaaaaa 2306
<210> 10
   <211> 2340
   <212> DNA
   <213> Homo sapiens
<400> 10
   gccccgcgcg ccgccggagt tccgcggagt tgaccgggtc ggcgccgtcg gtcctgagcg 60
   ctgccttccg cgttccgccg cggccccacc tggagccccc gccccgcgcc atggccggag 120
   ctcccggggc gcagcgctga cggcggcggg gggagcgcgc catgcccagc aggaccggcc 180
   ccaagatgga agggagcggc ggccgcgtcc gcctcaaggc gcattacggg ggggacatct 240
   tcatcaccag cgtggacgcc gccacgacct tcgaggagct ctgtgaggaa gtgagagaca 300
   tgtgtcgtct gcaccagcag cacccgctca ccctcaagtg ggtggacagc gaaggtgacc 360
   cttgcacggt gtcctcccag atggagctgg aagaggcttt ccgcctggcc cgtcagtgca 420
   gggatgaagg cctcatcatt catgttttcc cgagcacccc tgagcagcct ggcctgccat 480
   gtccgggaga agacaaatct atctaccgcc ggggagccag aagatggagg aagctgtacc 540
   gtgccaacgg ccacctcttc caagccaagc gctttaacag gagagcgtac tgcggtcagt 600
   gcagcgagag gatatggggc ctcgcgaggc aaggctacag gtgcatcaac tgcaaactgc 660
   tggtccataa gcgctgccac ggcctcgtcc cgctgacctg caggaagcat atggattctg 720
   tcatgccttc ccaagagcct ccagtagacg acaagaacga ggacgccgac cttccttccg 780
   aggagacaga tggaattgct tacatttcct catcccggaa gcatgacagc attaaagacg 840
   actcggagga ccttaagcca gttatcgatg ggatggatgg aatcaaaatc tctcaggggc 900
   ttgggctgca ggactttgac ctaatcagag tcatcgggcg cgggagctac gccaaggttc 960
   tcctggtgcg gttgaagaag aatgaccaaa tttacgccat gaaagtggtg aagaaagagc 1020
   tggtgcatga tgacgaggat attgactggg tacagacaga gaagcacgtg tttgagcagg 1080
   catccagcaa ccccttcctg gtcggattac actcctgctt ccagacgaca agtcggttgt 1140
   tcctggtcat tgagtacgtc aacggcgggg acctgatgtt ccacatgcag aggcagagga   1200
   agctccctga ggagcacgcc aggttctacg cggccgagat ctgcatcgcc ctcaacttcc   1260
   tgcacgagag ggggatcatc tacagggacc tgaagctgga caacgtcctc ctggatgcgg   1320
   acgggcacat caagctcaca gactacggca tgtgcaagga aggcctgggc cctggtgaca   1380
   caacgagcac tttctgcgga accccgaatt acatcgcccc cgaaatcctg cggggagagg   1440
   agtacgggtt cagcgtggac tggtgggcgc tgggagtcct catgtttgag atgatggccg   1500
   ggcgctcccc gttcgacatc atcaccgaca acccggacat gaacacagag gactaccttt   1560
   tccaagtgat cctggagaag cccatccgga tcccccggtt cctgtccgtc aaagcctccc   1620
   atgttttaaa aggattttta aataaggacc ccaaagagag gctcggctgc cggccacaga   1680
   ctggattttc tgacatcaag tcccacgcgt tcttccgcag catagactgg gacttgctgg   1740
   agaagaagca ggcgctccct ccattccagc cacagatcac agacgactac ggtctggaca   1800
   actttgacac acagttcacc agcgagcccg tgcagctgac cccagacgat gaggatgcca   1860
   taaagaggat cgaccagtca gagttcgaag gctttgagta tatcaaccca ttattgctgt   1920
   ccaccgagga gtcggtgtga ggccgcgtgc gtctctgtcg tggacacgcg tgattgaccc   1980
   tttaactgta tccttaacca ccgcatatgc atgccaggct gggcacggct ccgagggcgg   2040
   ccagggacag acgcttgcgc cgagaccgca gagggaagcg tcagcgggcg ctgctgggag   2100
   cagaacagtc cctcacacct gggcccgggc aggccagctt cgtgctggag gaacttgctg   2160
   ctgttcctgc gtcgcggcgg atccgcgggg accctgccga gggggctgtc atgcggtttc   2220
   caaggtgcac attttccacg gaaacagaac tcgatgcact gacctgctcc gccaggaaag   2280
   tgagcgtgta gcgtcctgag gaataaaatg ttccgatgaa aaaaaaaaaa aaaaaaaaaa   2340
<210> 11
   <211> 587
   <212> PRT
   <213> Homo sapiens
<400> 11
   Met Ser His Thr Val Ala Gly Gly Gly Ser Gly Asp His Ser His Gln
   1               5                   10                  15
   Val Arg Val Lys Ala Tyr Tyr Arg Gly Asp Ile Met Ile Thr His Phe
               20                  25                  30
   Glu Pro Ser Ile Ser Phe Glu Gly Leu Cys Asn Glu Val Arg Asp Met
           35                  40                  45
   Cys Ser Phe Asp Asn Glu Gln Leu Phe Thr Met Lys Trp Ile Asp Glu
       50                  55                  60
   Glu Gly Asp Pro Cys Thr Val Ser Ser Gln Leu Glu Leu Glu Glu Ala
   65                  70                  75                  80
   Phe Arg Leu Tyr Glu Leu Asn Lys Asp Ser Glu Leu Leu Ile His Val
                   85                  90                  95
   Phe Pro Cys Val Pro Glu Arg Pro Gly Met Pro Cys Pro Gly Glu Asp
               100                 105                 110
   Lys Ser Ile Tyr Arg Arg Gly Ala Arg Arg Trp Arg Lys Leu Tyr Cys
           115                 120                 125
   Ala Asn Gly His Thr Phe Gln Ala Lys Arg Phe Asn Arg Arg Ala His
       130                 135                 140
   Cys Ala Ile Cys Thr Asp Arg Ile Trp Gly Leu Gly Arg Gln Gly Tyr
   145                 150                 155                 160
   Lys Cys Ile Asn Cys Lys Leu Leu Val His Lys Lys Cys His Lys Leu
                   165                 170                 175
   Val Thr Ile Glu Cys Gly Arg His Ser Leu Pro Gln Glu Pro Val Met
               180                 185                 190
   Pro Met Asp Gln Ser Ser Met His Ser Asp His Ala Gln Thr Val Ile
           195                 200                 205
   Pro Tyr Asn Pro Ser Ser His Glu Ser Leu Asp Gln Val Gly Glu Glu
       210                 215                 220
   Lys Glu Ala Met Asn Thr Arg Glu Ser Gly Lys Ala Ser Ser Ser Leu
   225                 230                 235                 240
   Gly Leu Gln Asp Phe Asp Leu Leu Arg Val Ile Gly Arg Gly Ser Tyr
                   245                 250                 255
   Ala Lys Val Leu Leu Val Arg Leu Lys Lys Thr Asp Arg Ile Tyr Ala
               260                 265                 270
   Met Lys Val Val Lys Lys Glu Leu Val Asn Asp Asp Glu Asp Ile Asp
   275 280 285
   Trp Val Gln Thr Glu Lys His Val Phe Glu Gln Ala Ser Asn His Pro
   290 295 300
   Phe Leu Val Gly Leu His Ser Cys Phe Gln Thr Glu Ser Arg Leu Phe
   305 310 315 320
   Phe Val Ile Glu Tyr Val Asn Gly Gly Asp Leu Met Phe His Met Gln
   325 330 335
   Arg Gln Arg Lys Leu Pro Glu Glu His Ala Arg Phe Tyr Ser Ala Glu
   340 345 350
   Ile Ser Leu Ala Leu Asn Tyr Leu His Glu Arg Gly Ile Ile Tyr Arg
   355 360 365
   Asp Leu Lys Leu Asp Asn Val Leu Leu Asp Ser Glu Gly His Ile Lys
   370 375 380
   Leu Thr Asp Tyr Gly Met Cys Lys Glu Gly Leu Arg Pro Gly Asp Thr
   385 390 395 400
   Thr Ser Thr Phe Cys Gly Thr Pro Asn Tyr Ile Ala Pro Glu Ile Leu
   405 410 415
   Arg Gly Glu Asp Tyr Gly Phe Ser Val Asp Trp Trp Ala Leu Gly Val
   420 425 430
   Leu Met Phe Glu Met Met Ala Gly Arg Ser Pro Phe Asp Ile Val Gly
   435 440 445
   Ser Ser Asp Asn Pro Asp Gln Asn Thr Glu Asp Tyr Leu Phe Gln Val
   450 455 460
   Ile Leu Glu Lys Gln Ile Arg Ile Pro Arg Ser Leu Ser Val Lys Ala
   465 470 475 480
   Ala Ser Val Leu Lys Ser Phe Leu Asn Lys Asp Pro Lys Glu Arg Leu
   485 490 495
   Gly Cys His Pro Gln Thr Gly Phe Ala Asp Ile Gln Gly His Pro Phe
   500 505 510
   Phe Arg Asn Val Asp Trp Asp Met Met Glu Gln Lys Gln Val Val Pro
   515 520 525
   Pro Phe Lys Pro Asn Ile Ser Gly Glu Phe Gly Leu Asp Asn Phe Asp
   530 535 540
   Ser Gln Phe Thr Asn Glu Pro Val Gln Leu Thr Pro Asp Asp Asp Asp
   545 550 555 560
   Ile Val Arg Lys Ile Asp Gln Ser Glu Phe Glu Gly Phe Glu Tyr Ile
   565 570 575
   Asn Pro Leu Leu Met Ser Ala Glu Glu Cys Val
   580 585
<210> 12
   <211> 592
   <212> PRT
   <213> Homo sapiens
<400> 12
   Met Pro Ser Arg Thr Asp Pro Lys Met Glu Gly Ser Gly Gly Arg Val
   1 5 10 15
   Arg Leu Lys Ala His Tyr Gly Gly Asp Ile Phe Ile Thr Ser Val Asp
   20 25 30
   Ala Ala Thr Thr Phe Glu Glu Leu Cys Glu Glu Val Arg Asp Met Cys
   35 40 45
   Arg Leu His Gln Gln His Pro Leu Thr Leu Lys Trp Val Asp Ser Glu
   50 55 60
   Gly Asp Pro Cys Thr Val Ser Ser Gln Met Glu Leu Glu Glu Ala Phe
   65 70 75 80
   Arg Leu Ala Arg Gln Cys Arg Asp Glu Gly Leu Ile Ile His Val Phe
   85 90 95
   Pro Ser Thr Pro Glu Gln Pro Gly Leu Pro Cys Pro Gly Glu Asp Lys
   100 105 110
   Ser Ile Tyr Arg Arg Gly Ala Arg Arg Trp Arg Lys Leu Tyr Arg Ala
   115 120 125
   Asn Gly His Leu Phe Gln Ala Lys Arg Phe Asn Arg Arg Ala Tyr Cys
   130 135 140
   Gly Gln Cys Ser Glu Arg Ile Trp Gly Leu Ala Arg Gln Gly Tyr Arg
   145 150 155 160
   Cys Ile Asn Cys Lys Leu Leu Val His Lys Arg Cys His Gly Leu Val
   165 170 175
   Pro Leu Thr Cys Arg Lys His Met Asp Ser Val Met Pro Ser Gln Glu
   180 185 190
   Pro Pro Val Asp Asp Lys Asn Glu Asp Ala Asp Leu Pro Ser Glu Glu
   195 200 205
   Thr Asp Gly Ile Ala Tyr Ile Ser Ser Ser Arg Lys His Asp Ser Ile
   210 215 220
   Lys Asp Asp Ser Glu Asp Leu Lys Pro Val Ile Asp Gly Met Asp Gly
   225 230 235 240
   Ile Lys Ile Ser Gln Gly Leu Gly Leu Gln Asp Phe Asp Leu Ile Arg
   245 250 255
   Val Ile Gly Arg Gly Ser Tyr Ala Lys Val Leu Leu Val Arg Leu Lys
   260 265 270
   Lys Asn Asp Gln Ile Tyr Ala Met Lys Val Val Lys Lys Glu Leu Val
   275 280 285
   His Asp Asp Glu Asp Ile Asp Trp Val Gln Thr Glu Lys His Val Phe
   290 295 300
   Glu Gln Ala Ser Ser Asn Pro Phe Leu Val Gly Leu His Ser Cys Phe
   305 310 315 320
   Gln Thr Thr Ser Arg Leu Phe Leu Val Ile Glu Tyr Val Asn Gly Gly
   325 330 335
   Asp Leu Met Phe His Met Gln Arg Gln Arg Lys Leu Pro Glu Glu His
               340                 345                 350
   Ala Arg Phe Tyr Ala Ala Glu Ile Cys Ile Ala Leu Asn Phe Leu His
           355                 360                 365
   Glu Arg Gly Ile Ile Tyr Arg Asp Leu Lys Leu Asp Asn Val Leu Leu
       370                 375                 380
   Asp Ala Asp Gly His Ile Lys Leu Thr Asp Tyr Gly Met Cys Lys Glu
   385                 390                 395                 400
   Gly Leu Gly Pro Gly Asp Thr Thr Ser Thr Phe Cys Gly Thr Pro Asn
                   405                 410                 415
   Tyr Ile Ala Pro Glu Ile Leu Arg Gly Glu Glu Tyr Gly Phe Ser Val
               420                 425                 430
   Asp Trp Trp Ala Leu Gly Val Leu Met Phe Glu Met Met Ala Gly Arg
           435                 440                 445
   Ser Pro Phe Asp Ile Ile Thr Asp Asn Pro Asp Met Asn Thr Glu Asp
       450                 455                 460
   Tyr Leu Phe Gln Val Ile Leu Glu Lys Pro Ile Arg Ile Pro Arg Phe
   465                 470                 475                 480
   Leu Ser Val Lys Ala Ser His Val Leu Lys Gly Phe Leu Asn Lys Asp
                   485                 490                 495
   Pro Lys Glu Arg Leu Gly Cys Arg Pro Gln Thr Gly Phe Ser Asp Ile
               500                 505                 510
   Lys Ser His Ala Phe Phe Arg Ser Ile Asp Trp Asp Leu Leu Glu Lys
           515                 520                 525
   Lys Gln Ala Leu Pro Pro Phe Gln Pro Gln Ile Thr Asp Asp Tyr Gly
       530                 535                 540
   Leu Asp Asn Phe Asp Thr Gln Phe Thr Ser Glu Pro Val Gln Leu Thr
   545                 550                 555                 560
   Pro Asp Asp Glu Asp Ala Ile Lys Arg Ile Asp Gln Ser Glu Phe Glu
                   565                 570                 575
   Gly Phe Glu Tyr Ile Asn Pro Leu Leu Leu Ser Thr Glu Glu Ser Val
               580                 585                 590

## Description

### BACKGROUND OF THE INVENTION

The *Drosophila Melanogaster* Armadillo/beta-catenin protein is implicated in multiple cellular functions. The protein functions in cell signaling via the Wingless (Wg)/Wnt signaling pathway. It also functions as a cell adhesion protein at the cell membrane in a complex with E-cadherin and alpha-catenin (Cox et al. (1996) J. Cell Biol. 134: 133-148; Godt and Tepass (1998) Nature 395: 387-391; White et al. (1998) J Cell biol. 140:183-195). These two roles of beta -catenin can be separated from each other (Orsulic and Peifer (1996) J. Cell Biol. 134: 1283-1300; Sanson et al. (1996) Nature 383: 627-630).

In Wingless cell signaling, beta -catenin levels are tightly regulated by a complex containing APC, Axin, and GSK3 beta /SGG/ZW3 (Peifer et al. (1994) Development 120: 369-380).

The Wingless/ beta -catenin signaling pathway is frequently mutated in human cancers, particularly those of the colon. Mutations in the tumor suppressor gene APC, as well as point mutations in beta -catenin itself lead to the stabilization of the beta -catenin protein and inappropriate activation of this pathway.

The protein kinase C (PKC) family of serine/threonine protein kinases has at least eight members, which are differentially expressed and are involved in a wide variety of cellular processes such as proliferation, differentiation and secretion. Protein kinase C iota (PRKCI) belongs to the PKC family, and is calcium-independent and phospholipid-dependent. PRKCI can be recruited to vesicle tubular clusters (VTCs) by direct interaction with the small GTPase RAB2, where this kinase phosphorylates glyceraldehydes-3-phosphate dehydrogenase (GAPD/GAPDH) and plays a role in microtubule dynamics in the early secretory pathway. PRKCI is necessary for BCL-ABL-mediated resistance to drug-induced apoptosis and therefore protects leukemia cells against drug-induced apoptosis.

Protein kinase C (PKC) zeta is another member of the PKC family of serine/threonine kinases. Unlike the classical PKC isoenzymes which are calcium-dependent, PKC zeta exhibits a constitutive kinase activity which is independent of calcium. Mice with targeted disruption of PRKCZ gene are known in the art (Leitges M et al (2001) Molec Cell 8:771-780).

The ability to manipulate the genomes of model organisms such as *Drosophila* provides a powerful means to analyze biochemical processes that, due to significant evolutionary conservation, have direct relevance to more complex vertebrate organisms. Due to a high level of gene and pathway conservation, the strong similarity of cellular processes, and the functional conservation of genes between these model organisms and mammals, identification of the involvement of novel genes in particular pathways and their functions in such model organisms can directly contribute to the understanding of the correlative pathways and methods of modulating them in mammals (see, for example, Mechler BM et al., 1985 EMBO J 4:1551-1557; Gateff E. 1982 Adv. Cancer Res. 37: 33-74; Watson KL., et al., 1994 J Cell Sci. 18: 19-33; Miklos GL, and Rubin GM. 1996 Cell 86:521-529; Wassarman DA, et al., 1995 Curr Opin Gen Dev 5: 44-50; and Booth DR. 1999 Cancer Metastasis Rev. 18: 261-284). For example, a genetic screen can be carried out in an invertebrate model organism having underexpression (e.g. knockout) or overexpression of a gene (referred to as a "genetic entry point") that yields a visible phenotype. Additional genes are mutated in a random or targeted manner. When a gene mutation changes the original phenotype caused by the mutation in the genetic entry point, the gene is identified as a "modifier" involved in the same or overlapping pathway as the genetic entry point. When the genetic entry point is an ortholog of a human gene implicated in a disease pathway, such as beta catenin, modifier genes can be identified that may be attractive candidate targets for novel therapeutics.

Williams Carol, et al., Int. J Oncology (2001), vol. 19, no. 6, pp 1227-1233 reports reduced expression of Wnt-1 and E-cadherin and diminished beta-catenin stability in MCF-7 breast cancer cells that overexpress protein kinase C-alpha.

Ren Jian, et al., J Biol Chem. (2002), vol. 277, no. 20, pp 17616-17622 reports that protein kinase C delta regulates function of the DF3/MUC1 carcinoma antigen in beta-catenin signaling.

WO 02/058450 discloses a method of identifying a compound that inhibits (a) the physical interaction (binding) between MUC1 and tumor progressors (e.g., beta-catening, c-Src, EGF-R, p120^{ctn}, or PKC delta) and/or (b) phosphorylation of MUC1 by tumor progressors with kinase activity (e.g., c-Src, EGF-R, or PKC delta). There is also disclosed a method of inhibiting an interaction between MUC1 and beta-catening and a method of inhibiting expression of MUC1 or a tumor progressor in a cell.

WO 03/023048 discloses methods of diagnosing diseases and conditions associated with PKC lambda, methods for identifying compounds that can be used to treat or to prevent such diseases and conditions, and methods of using these compounds to treat or to prevent such diseases and conditions.

WO 03/052068 discloses that human MBCAT genes are modulators of the beta-catenin pathway, and thus are therapeutic targets for disorders associated with defective beta-catenin function. Methods for identifying modulators of beta-catenin, comprising screening for agents that modulate the activity of MBCAT are provided.

US 2002/015943 discloses that truncations of the tumour suppressor Adenomatous polyposis coli (APC) which are found in cancer cells cause high levels of nuclear beta-catenin to accumulate by 'trapping' beta-catenin within the nucleus.

Chen R, et al., J Biol Chem. (2000), vol. 275, pp 17894-17899 discusses that Wnt signalling to b-catenin involves two interactive components.

Monga S, et al., Gastroenterology (2003), vol. 124, pp 202-216 discusses the role of beta-catenin antisense studies in embryonic liver cultures in proliferation, apoptosis and lineage specification.

### SUMMARY OF THE INVENTION

We have discovered genes that modify the beta catenin pathway in *Drosophila,* and identified their human orthologs, hereinafter referred to as Protein Kinase C (PRKC). The invention provides methods for utilizing these beta catenin modifier genes and polypeptides to identify PRKC-modulating agents that are candidate therapeutic agents that can be used in the treatment of disorders associated with defective or impaired beta catenin function and/or PRKC function. Preferred PRKC-modulating agents specifically bind to PRKC polypeptides and restore beta catenin function. Other preferred PRKC-modulating agents are nucleic acid modulators such as antisense oligomers and RNAi that repress PRKC gene expression or product activity by, for example, binding to and inhibiting the respective nucleic acid (i.e. DNA or mRNA).

PRKC modulating agents may be evaluated by any convenient *in vitro* or *in vivo* assay for molecular interaction with a PRKC polypeptide or nucleic acid. In one embodiment, candidate PRKC modulating agents are tested with an in vitro assay system capable of detecting PRKC expression and/or kinase activity, comprising a PRKC polypeptide or nucleic acid of SEQ ID NOs: 1, 2, 4 or 5. Agents that produce a change in the activity of the assay system relative to controls are identified as candidate beta catenin modulating agents. The assay system may be cell-based or cell-free. PRKC-modulating agents include PRKC related proteins (e.g. dominant negative mutants, and biotherapeutics); PRKC -specific antibodies; PRKC -specific antisense oligomers and other nucleic acid modulators; and chemical agents that specifically bind to or interact with PRKC or compete with PRKC binding partner (e.g. by binding to a PRKC binding partner). In one specific embodiment, a small molecule modulator is identified using a kinase assay. In specific embodiments, the screening assay system is a binding assay.

In another embodiment, candidate beta catenin pathway modulating agents are further tested using an in vitro second assay system that detects changes in the beta catenin pathway, such as angiogenic, apoptotic, or cell proliferation changes produced by the originally identified candidate agent or an agent derived from the original agent, comprising cultured cells expressing PRKC.

The invention further provides an in vitro method for modulating the the beta catenin pathway in a mammalian cell by contacting the mammalian cell with an agent that specifically modulates a PRKC polypeptide or nucleic acid of SEQ ID NOs, 1, 2, 4 or 5, wherein said PRKC polypeptide is a PRKC protein encoded by said PRKC nucleic acid, or a fragment or derivative of said PRKC protein, and wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

In another embodiment, there is provided an agent that specifically modulates: (i) the expression of PRKC nucleic acid or polypeptide; and/or (ii) PRKC polypeptide kinase activity; for use in the treatment of a disease or disorder selected from an angiogenic, apoptotic and cell proliferation disease or disorder, wherein said PRKC nucleic acid has a nucleotide sequence shown as Sequence ID Nos: 1, 2, 4 or 5, wherein said PRKC polypeptide is a PRKC protein encoded by said PRKC nucleic acid, or a fragment or derivative of said PRKC protein, and wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

In a further embodiment, there is provided the use of an agent that specifically modulates (i) expression of PRKC nucleic acid or polypeptide and/or (ii) PRKC polypeptide kinase activity; in the preparation of a medicament for the treatment of a disease or disorder selected from an angiogenic, apoptotic and cell proliferation disease or disorder; wherein said PRKC nucleic acid has a nucleotide sequence shown as Sequence ID Nos: 1, 2, 4 or 5; wherein said PRKC polypeptide is a PRKC protein encoded by said PRKC nucleic acid, or a fragment or derivative of said PRKC protein; and wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

In yet a further embodiment, the present invention provides an in vitro method for diagnosing a disorder or disease associated with a defect in the beta catenin pathway in a biological sample from a patient comprising:
(a) contacting the sample with a probe for PRKC nucleic acid or polypeptide expression, wherein said PRKC nucleic acid has a nucleotide sequence shown as Sequence ID Nos: 1, 2, 4 or 5 and wherein said PRKC polypeptide is a PRKC protein encoded by said PRKC nucleic acid, or a fragment or derivative of said PRKC protein;
(b) comparing results from step (a) with a control; and
(c) determining whether step (b) indicates a likelihood of a disorder or disease associated with a defect in the beta catenin pathway, wherein the disease or disorder is selected from an angiogenic, apoptotic and cell proliferation disease or disorder.

### DETAILED DESCRIPTION OF THE INVENTION

In a screen to identify enhancers and suppressors of the Wg signaling pathway, we generated activated beta -catenin models in *Drosophila* based on human tumor data (Polakis (2000) Genes and Development 14: 1837-1851). We identified modifiers of the Wg pathway and identified their orthologs. The APKC gene was identified as a modifier of the beta catenin pathway. Accordingly, vertebrate orthologs of these modifiers, and preferably the human orthologs, PRKC genes (i.e., nucleic acids and polypeptides) are attractive drug targets for the treatment of pathologies associated with a defective beta catenin signaling pathway, such as cancer.

Modulation of the PRKC or their respective binding partners is useful for understanding the association of the beta catenin pathway and its members in normal and disease conditions and for developing diagnostics and therapeutic modalities for beta catenin related pathologies. PRKC-modulating agents that act by inhibiting or enhancing PRKC expression, directly or indirectly, for example, by affecting a PRKC function such as enzymatic (e.g., catalytic) or binding activity, can be identified using methods provided herein. PRKC modulating agents are useful in diagnosis, therapy and pharmaceutical development.

### Nucleic acids and polypeptides of the invention

Sequences related to PRKC nucleic acids and polypeptides that can be used in the invention are disclosed in Genbank (referenced by Genbank identifier (GI) number) as GI#s 4506070 (SEQ ID NO:1), 18314568 (SEQ ID NO:2), 34222297 (SEQ ID NO:4), 34191041 (SEQ ID NO:5) for nucleic acids, and polypeptides encoded by those nucleic acids. Also disclosed herein, although not part of the present invention, are GI#s 10864649 (SEQ ID NO:6), 14165514 (SEQ ID NO:7), 307355 (SEQ ID NO:8), 33873791 (SEQ ID NO:9), and 33878518 (SEQ ID NO:10) for nucleic acid, and GI#s 4506071 (SEQ ID NO:11) and 10864650 (SEQ ID NO:12) for polypeptides. Additionally, nucleic acid of SEQ ID NO:3 is also disclosed.

The term "PRKC polypeptide" refers to a full-length PRKC protein or a functionally active fragment or derivative thereof. A "functionally active" PRKC fragment or derivative exhibits one or more functional activities associated with a full-length, wild-type PRKC protein, such as antigenic or immunogenic activity, enzymatic activity, ability to bind natural cellular substrates, etc. The functional activity of PRKC proteins, derivatives and fragments can be assayed by various methods known to one skilled in the art (Current Protocols in Protein Science (1998) Coligan et al., eds., John Wiley & Sons, Inc., Somerset, New Jersey) and as further discussed below. In one embodiment, a functionally active PRKC polypeptide is a PRKC derivative capable of rescuing defective endogenous PRKC activity, such as in cell based or animal assays; the rescuing derivative may be from the same or a different species. For purposes herein, functionally active fragments also include those fragments that comprise one or more structural domains of a PRKC, such as a kinase domain or a binding domain. Protein domains can be identified using the PFAM program (Bateman A., et al., Nucleic Acids Res, 1999, 27:260-2). For example, the kinase domain (PFAM 00069) of PRKC from GI#s 4506071 and 10864650 (SEQ ID NOs:11 and 12, respectively) is located respectively at approximately amino acid residues 245 to 513 and 252 to 518. Further, the Kinase C terminal domain (PFAM 00433) of the same proteins is located respectively at approximately amino acid residues 514 to 580 and 519 to 585. Methods for obtaining PRKC polypeptides are also further described below. In some embodiments, preferred fragments are functionally active, domain-containing fragments comprising at least 25 contiguous amino acids, preferably at least 50, more preferably 75, and most preferably at least 100 contiguous amino acids of a PRKC. In further preferred embodiments, the fragment comprises the entire functionally active domain.

The term "PRKC nucleic acid" refers to a DNA or RNA molecule that encodes a PRKC polypeptide. Preferably, the PRKC polypeptide or nucleic acid or fragment thereof is from a human, but can also be an ortholog, or derivative thereof with at least 70% sequence identity, preferably at least 80%, more preferably 85%, still more preferably 90%, and most preferably at least 95% sequence identity with human PRKC. Methods of identifying orthlogs are known in the art. Normally, orthologs in different species retain the same function, due to presence of one or more protein motifs and/or 3-dimensional structures. Orthologs are generally identified by sequence homology analysis, such as BLAST analysis, usually using protein bait sequences. Sequences are assigned as a potential ortholog if the best hit sequence from the forward BLAST result retrieves the original query sequence in the reverse BLAST (Huynen MA and Bork P, Proc Natl Acad Sci (1998) 95:5849-5856; Huynen MA et al., Genome Research (2000) 10:1204-1210). Programs for multiple sequence alignment, such as CLUSTAL (Thompson JD et al, 1994, Nucleic Acids Res 22:4673-4680) may be used to highlight conserved regions and/or residues of orthologous proteins and to generate phylogenetic trees. In a phylogenetic tree representing multiple homologous sequences from diverse species (e.g., retrieved through BLAST analysis), orthologous sequences from two species generally appear closest on the tree with respect to all other sequences from these two species. Structural threading or other analysis of protein folding (e.g., using software by ProCeryon, Biosciences, Salzburg, Austria) may also identify potential orthologs. In evolution, when a gene duplication event follows speciation, a single gene in one species, such as *Drosophila,* may correspond to multiple genes (paralogs) in another, such as human. As used herein, the term "orthologs" encompasses paralogs. As used herein, "percent (%) sequence identity" with respect to a subject sequence, or a specified portion of a subject sequence, is defined as the percentage of nucleotides or amino acids in the candidate derivative sequence identical with the nucleotides or amino acids in the subject sequence (or specified portion thereof), after aligning the sequences and introducing gaps, if necessary to achieve the maximum percent sequence identity, as generated by the program WU-BLAST-2.0a19 (Altschul et al., J. Mol. Biol. (1997) 215:403-410) with all the search parameters set to default values. The HSP S and HSP S2 parameters are dynamic values and are established by the program itself depending upon the composition of the particular sequence and composition of the particular database against which the sequence of interest is being searched. A % identity value is determined by the number of matching identical nucleotides or amino acids divided by the sequence length for which the percent identity is being reported. "Percent (%) amino acid sequence similarity" is determined by doing the same calculation as for determining % amino acid sequence identity, but including conservative amino acid substitutions in addition to identical amino acids in the computation.

A conservative amino acid substitution is one in which an amino acid is substituted for another amino acid having similar properties such that the folding or activity of the protein is not significantly affected. Aromatic amino acids that can be substituted for each other are phenylalanine, tryptophan, and tyrosine; interchangeable hydrophobic amino acids are leucine, isoleucine, methionine, and valine; interchangeable polar amino acids are glutamine and asparagine; interchangeable basic amino acids are arginine, lysine and histidine; interchangeable acidic amino acids are aspartic acid and glutamic acid; and interchangeable small amino acids are alanine, serine, threonine, cysteine and glycine.

Alternatively, an alignment for nucleic acid sequences is provided by the local homology algorithm of Smith and Waterman (Smith and Waterman, 1981, Advances in Applied Mathematics 2:482-489; database: European Bioinformatics Institute; Smith and Waterman, 1981, J. of Molec.Biol., 147:195-197; Nicholas et al., 1998, "A Tutorial on Searching Sequence Databases and Sequence Scoring Methods" (www.psc.edu) and references cited therein.; W.R. Pearson, 1991, Genomics 11:635-650). This algorithm can be applied to amino acid sequences by using the scoring matrix developed by Dayhoff (Dayhoff: Atlas of Protein Sequences and Structure, M. O. Dayhoff ed., 5 suppl. 3:353-358, National Biomedical Research Foundation, Washington, D.C., USA), and normalized by Gribskov (Gribskov 1986 Nucl. Acids Res. 14(6):6745-6763). The Smith-Waterman algorithm may be employed where default parameters are used for scoring (for example, gap open penalty of 12, gap extension penalty of two). From the data generated, the "Match" value reflects "sequence identity."

Derivative nucleic acid molecules of the subject nucleic acid molecules include sequences that hybridize to the nucleic acid sequence of a PRKC. The stringency of hybridization can be controlled by temperature, ionic strength, pH, and the presence of denaturing agents such as formamide during hybridization and washing. Conditions routinely used are set out in readily available procedure texts (*e*.*g*., Current Protocol in Molecular Biology, Vol. 1, Chap. 2.10, John Wiley & Sons, Publishers (1994); Sambrook et al., Molecular Cloning, Cold Spring Harbor (1989)). As discussed, a nucleic acid molecule is capable of hybridizing to a nucleic acid molecule containing the nucleotide sequence of a PRKC under high stringency hybridization conditions that are: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1h in a solution containing 0.1X SSC and 0.1% SDS (sodium dodecyl sulfate).

Alternatively, moderately stringent hybridization conditions are used that are: pretreatment of filters containing nucleic acid for 6 h at 40° C in a solution containing 35% formamide, 5X SSC, 50 mM Tris-HCl (pH7.5), 5mM EDTA, 0.1% PVP, 0.1% Ficoll, 1% BSA, and 500 µg/ml denatured salmon sperm DNA; hybridization for 18-20h at 40° C in a solution containing 35% formamide, 5X SSC, 50 mM Tris-HCl (pH7.5), 5mM EDTA, 0.02% PVP, 0.02% Ficoll, 0.2% BSA, 100 µg/ml salmon sperm DNA, and 10% (wt/vol) dextran sulfate; followed by washing twice for 1 hour at 55° C in a solution containing 2X SSC and 0.1% SDS.

Alternatively, low stringency conditions can be used that are: incubation for 8 hours to overnight at 37° C in a solution comprising 20% formamide, 5 x SSC, 50 mM sodium phosphate (pH 7.6), 5X Denhardt's solution, 10% dextran sulfate, and 20 µg/ml denatured sheared salmon sperm DNA; hybridization in the same buffer for 18 to 20 hours; and washing of filters in 1 x SSC at about 37° C for 1 hour.

### Isolation, Production, Expression, and Mis-expression of PRKC Nucleic Acids and Polypeptides

PRKC nucleic acids and polypeptides are useful for identifying and testing agents that modulate PRKC function and for other applications related to the involvement of PRKC in the beta catenin pathway. PRKC nucleic acids and derivatives and orthologs thereof may be obtained using any available method. For instance, techniques for isolating cDNA or genomic DNA sequences of interest by screening DNA libraries or by using polymerase chain reaction (PCR) are well known in the art. In general, the particular use for the protein will dictate the particulars of expression, production, and purification methods. For instance, production of proteins for use in screening for modulating agents may require methods that preserve specific biological activities of these proteins, whereas production of proteins for antibody generation may require structural integrity of particular epitopes. Expression of proteins to be purified for screening or antibody production may require the addition of specific tags (*e*.*g*., generation of fusion proteins). Overexpression of a PRKC protein for assays used to assess PRKC function, such as involvement in cell cycle regulation or hypoxic response, may require expression in eukaryotic cell lines capable of these cellular activities. Techniques for the expression, production, and purification of proteins are well known in the art; any suitable means therefore may be used (e.g., Higgins SJ and Hames BD (eds.) Protein Expression: A Practical Approach, Oxford University Press Inc., New York 1999; Stanbury PF et al., Principles of Fermentation Technology, 2nd edition, Elsevier Science, New York, 1995; Doonan S (ed.) Protein Purification Protocols, Humana Press, New Jersey, 1996; Coligan JE et al, Current Protocols in Protein Science (eds.), 1999, John Wiley & Sons, New York). In particular embodiments, recombinant PRKC is expressed in a cell line known to have defective beta catenin function. The recombinant cells are used in cell-based screening assay systems of the invention, as described further below.

The nucleotide sequence encoding a PRKC polypeptide can be inserted into any appropriate expression vector. The necessary transcriptional and translational signals, including promoter/enhancer element, can derive from the native PRKC gene and/or its flanking regions or can be heterologous. A variety of host-vector expression systems may be utilized, such as mammalian cell systems infected with virus (*e*.*g*. vaccinia virus, adenovirus, *etc*.); insect cell systems infected with virus (*e*.*g*. baculovirus); microorganisms such as yeast containing yeast vectors, or bacteria transformed with bacteriophage, plasmid, or cosmid DNA. An isolated host cell strain that modulates the expression of, modifies, and/or specifically processes the gene product may be used.

To detect expression of the PRKC gene product, the expression vector can comprise a promoter operably linked to a PRKC gene nucleic acid, one or more origins of replication, and, one or more selectable markers (*e*.*g*. thymidine kinase activity, resistance to antibiotics, *etc*.). Alternatively, recombinant expression vectors can be identified by assaying for the expression of the PRKC gene product based on the physical or functional properties of the PRKC protein in *in vitro* assay systems (*e*.*g*. immunoassays).

The PRKC protein, fragment, or derivative may be optionally expressed as a fusion, or chimeric protein product (i.e. it is joined via a peptide bond to a heterologous protein sequence of a different protein), for example to facilitate purification or detection. A chimeric product can be made by ligating the appropriate nucleic acid sequences encoding the desired amino acid sequences to each other using standard methods and expressing the chimeric product. A chimeric product may also be made by protein synthetic techniques, *e*.*g*. by use of a peptide synthesizer (Hunkapiller et al., Nature (1984) 310:105-111).

Once a recombinant cell that expresses the PRKC gene sequence is identified, the gene product can be isolated and purified using standard methods (*e*.*g*. ion exchange, affinity, and gel exclusion chromatography; centrifugation; differential solubility; electrophoresis). Alternatively, native PRKC proteins can be purified from natural sources, by standard methods (*e*.*g*. immunoaffinity purification). Once a protein is obtained, it may be quantified and its activity measured by appropriate methods, such as immunoassay, bioassay, or other measurements of physical properties, such as crystallography.

The methods of this invention may also use cells that have been engineered for altered expression (mis-expression) of PRKC or other genes associated with the beta catenin pathway. As used herein, mis-expression encompasses ectopic expression, overexpression, under-expression, and non-expression (*e*.*g*. by gene knock-out or blocking expression that would otherwise normally occur).

### General Information onGenetically modified animals

Animal models that have been genetically modified to alter PRKC expression may be used in *in vivo* assays to test for activity of a candidate beta catenin modulating agent, or to further assess the role of PRKC in a beta catenin pathway process such as apoptosis or cell proliferation. Preferably, the altered PRKC expression results in a detectable phenotype, such as decreased or increased levels of cell proliferation, angiogenesis, or apoptosis compared to control animals having normal PRKC expression. The genetically modified animal may additionally have altered beta catenin expression (e.g. beta catenin knockout). Preferred genetically modified animals are mammals such as primates, rodents (preferably mice or rats), among others. Preferred non-mammalian species include zebrafish, *C. elegans*, and *Drosophila.* Preferred genetically modified animals are transgenic animals having a heterologous nucleic acid sequence present as an extrachromosomal element in a portion of its cells, i.e. mosaic animals (see, for example, techniques described by Jakobovits, 1994, Curr. Biol. 4:761-763.) or stably integrated into its germ line DNA (i.e., in the genomic sequence of most or all of its cells). Heterologous nucleic acid is introduced into the germ line of such transgenic animals by genetic manipulation of, for example, embryos or embryonic stem cells of the host animal.

Methods of making transgenic animals are well-known in the art (for transgenic mice see Brinster et al., Proc. Nat. Acad. Sci. USA 82: 4438-4442 (1985), U.S. Pat. Nos. 4,736,866 and 4,870,009, both by Leder et al., U.S. Pat. No. 4,873,191 by Wagner et al., and Hogan, B., Manipulating the Mouse Embryo, Cold Spring Harbor Laboratory Press, Cold Spring Harbor, N.Y., (1986); for particle bombardment see U.S. Pat. No., 4,945,050, by Sandford *et al*.; for transgenic *Drosophila* see Rubin and Spradling, Science (1982) 218:348-53 and U.S. Pat. No. 4,670,388; for transgenic insects see Berghammer A.J. et al., A Universal Marker for Transgenic Insects (1999) Nature 402:370-371; for transgenic Zebrafish see Lin S., Transgenic Zebrafish, Methods Mol Biol. (2000);136:375-3830); for microinjection procedures for fish, amphibian eggs and birds see Houdebine and Chourrout, Experientia (1991) 47:897-905; for transgenic rats see Hammer et al., Cell (1990) 63:1099-1112; and for culturing of embryonic stem (ES) cells and the subsequent production of transgenic animals by the introduction of DNA into ES cells using methods such as electroporation, calcium phosphate/DNA precipitation and direct injection see, e.g., Teratocarcinomas and Embryonic Stem Cells, A Practical Approach, E. J. Robertson, ed., IRL Press (1987)). Clones of the nonhuman transgenic animals can be produced according to available methods (see Wilmut, I. et al. (1997) Nature 385:810-813; and PCT International Publication Nos. WO 97/07668 and WO 97/07669).

The transgenic animal may be a "knock-out" animal having a heterozygous or homozygous alteration in the sequence of an endogenous PRKC gene that results in a decrease of PRKC function, preferably such that PRKC expression is undetectable or insignificant. Knock-out animals are typically generated by homologous recombination with a vector comprising a transgene having at least a portion of the gene to be knocked out. Typically a deletion, addition or substitution has been introduced into the transgene to functionally disrupt it. The transgene can be a human gene (e.g., from a human genomic clone) but more preferably is an ortholog of the human gene derived from the transgenic host species. For example, a mouse PRKC gene is used to construct a homologous recombination vector suitable for altering an endogenous PRKC gene in the mouse genome. Detailed methodologies for homologous recombination in mice are available (see Capecchi, Science (1989) 244:1288-1292; Joyner et al., Nature (1989) 338:153-156). Procedures for the production of non-rodent transgenic mammals and other animals are also available (Houdebine and Chourrout, *supra*; Pursel et al., Science (1989) 244:1281-1288; Simms et al., Bio/Technology (1988) 6:179-183).

Knock-out animals, such as mice harboring a knockout of a specific gene, may be used to produce antibodies against the human counterpart of the gene that has been knocked out (Claesson MH et al., (1994) Scan J Immunol 40:257-264; Declerck PJ et al., (1995) J Biol Chem. 270:8397-400).

The transgenic animal may be "knock-in" animal having an alteration in its genome that results in altered expression (e.g., increased (including ectopic) or decreased expression) of the PRKC gene, e.g., by introduction of additional copies of PRKC, or by operatively inserting a regulatory sequence that provides for altered expression of an endogenous copy of the PRKC gene. Such regulatory sequences include inducible, tissue-specific, and constitutive promoters and enhancer elements. The knock-in can be homozygous or heterozygous.

Transgenic nonhuman animals can also be produced that contain selected systems allowing for regulated expression of the transgene. One example of such a system that may be produced is the cre/loxP recombinase system of bacteriophage P1 (Lakso et al., PNAS (1992) 89:6232-6236; U.S. Pat. No. 4,959,317). If a cre/loxP recombinase system is used to regulate expression of the transgene, animals containing transgenes encoding both the Cre recombinase and a selected protein are required. Such animals can be provided through the construction of "double" transgenic animals, e.g., by mating two transgenic animals, one containing a transgene encoding a selected protein and the other containing a transgene encoding a recombinase. Another example of a recombinase system is the FLP recombinase system of Saccharomyces cerevisiae (O'Gorman et al. (1991) Science 251:1351-1355; U.S. Pat. No. 5,654,182). Both Cre-LoxP and Flp-Frt may be used in the same system to regulate expression of the transgene, and for sequential deletion of vector sequences in the same cell (Sun X et al (2000) Nat Genet 25:83-6).

### Modulating Agents

The invention provides methods to identify agents that interact with and/or modulate the expression and/or kinase activity of PRKC and/or the beta catenin pathway. Modulating agents identified by the methods are also part of the invention. Such agents are useful in a variety of diagnostic and therapeutic applications associated with the beta catenin pathway, as well as in further analysis of the PRKC protein and its contribution to the beta catenin pathway. Accordingly, the invention also provides an in vitro method for modulating the beta catenin pathway comprising the step of specifically modulating PRKC expression and/or kinase activity by administering a PRKC-interacting or -modulating agent.

As used herein, a "PRKC-modulating agent" is any agent that modulates PRKC function, for example, an agent that interacts with PRKC to inhibit or enhance PRKC activity or otherwise affect normal PRKC function. PRKC function can be affected at any level, including transcription, protein expression, protein localization, and cellular or extra-cellular activity. In a preferred embodiment, the PRKC - modulating agent specifically modulates the function of the PRKC. The phrases "specific modulating agent", "specifically modulates", etc., are used herein to refer to modulating agents that directly bind to the PRKC polypeptide or nucleic acid, and preferably inhibit, enhance, or otherwise alter, the function of the PRKC. These phrases also encompass modulating agents that alter the interaction of the PRKC with a binding partner, substrate, or cofactor (e.g. by binding to a binding partner of a PRKC, or to a protein/binding partner complex, and altering PRKC function). In a further preferred embodiment, the PRKC- modulating agent is a modulator of the beta catenin pathway (e.g. it restores and/or upregulates beta catenin function) and thus is also a beta catenin-modulating agent.

Preferred PRKC-modulating agents include small molecule compounds; PRKC-interacting proteins, including antibodies and other biotherapeutics; and nucleic acid modulators such as antisense and RNA inhibitors. The modulating agents may be formulated in pharmaceutical compositions, for example, as compositions that may comprise other active ingredients, as in combination therapy, and/or suitable carriers or excipients. Techniques for formulation and administration of the compounds may be found in "Remington's Pharmaceutical Sciences" Mack Publishing Co., Easton, PA, 19th edition.

### Small molecule modulators

Small molecules are often preferred to modulate function of proteins with enzymatic function, and/or containing protein interaction domains. Chemical agents, referred to in the art as "small molecule" compounds are typically organic, non-peptide molecules, having a molecular weight up to 10,000, preferably up to 5,000, more preferably up to 1,000, and most preferably up to 500 daltons. This class of modulators includes chemically synthesized molecules, for instance, compounds from combinatorial chemical libraries. Synthetic compounds may be rationally designed or identified based on known or inferred properties of the PRKC protein or may be identified by screening compound libraries. Alternative appropriate modulators of this class are natural products, particularly secondary metabolites from organisms such as plants or fungi, which can also be identified by screening compound libraries for PRKC-modulating activity. Methods for generating and obtaining compounds are well known in the art (Schreiber SL, Science (2000) 151: 1964-1969; Radmann J and Gunther J, Science (2000) 151:1947-1948).

Small molecule modulators identified from screening assays, as described below, can be used as lead compounds from which candidate clinical compounds may be designed, optimized, and synthesized. Such clinical compounds may have utility in treating pathologies associated with the beta catenin pathway. The activity of candidate small molecule modulating agents may be improved several-fold through iterative secondary functional validation, as further described below, structure determination, and candidate modulator modification and testing. Additionally, candidate clinical compounds are generated with specific regard to clinical and pharmacological properties. For example, the reagents may be derivatized and re-screened using *in vitro* and *in vivo* assays to optimize activity and minimize toxicity for pharmaceutical development.

### Protein Modulators

Specific PRKC-interacting proteins are useful in a variety of diagnostic and therapeutic applications related to the beta catenin pathway and related disorders, as well as in validation assays for other PRKC-modulating agents. In a preferred embodiment, PRKC-interacting proteins affect normal PRKC function, including transcription, protein expression, protein localization, and cellular or extra-cellular activity. In another embodiment, PRKC-interacting proteins are useful in detecting and providing information about the function of PRKC proteins, as is relevant to beta catenin related disorders, such as cancer (e.g., for diagnostic means).

An PRKC-interacting protein may be endogenous, i.e. one that naturally interacts genetically or biochemically with a PRKC, such as a member of the PRKC pathway that modulates PRKC expression, localization, and/or activity. PRKC-modulators include dominant negative forms of PRKC-interacting proteins and of PRKC proteins themselves. Yeast two-hybrid and variant screens offer preferred methods for identifying endogenous PRKC-interacting proteins (Finley, R. L. et al. (1996) in DNA Cloning-Expression Systems: A Practical Approach, eds. Glover D. & Hames B. D (Oxford University Press, Oxford, England), pp. 169-203; Fashema SF et al., Gene (2000) 250:1-14; Drees BL Curr Opin Chem Biol (1999) 3:64-70; Vidal M and Legrain P Nucleic Acids Res (1999) 27:919-29; and U.S. Pat. No. 5,928,868). Mass spectrometry is an alternative preferred method for the elucidation of protein complexes (reviewed in, e.g., Pandley A and Mann M, Nature (2000) 405:837-846; Yates JR 3rd, Trends Genet (2000) 16:5-8).

An PRKC-interacting protein may be an exogenous protein, such as a PRKC-specific antibody or a T-cell antigen receptor (see, e.g., Harlow and Lane (1988) Antibodies, A Laboratory Manual, Cold Spring Harbor Laboratory; Harlow and Lane (1999) Using antibodies: a laboratory manual. Cold Spring Harbor, NY: Cold Spring Harbor Laboratory Press). PRKC antibodies are further discussed below.

In preferred embodiments, a PRKC-interacting protein specifically binds a PRKC protein. In alternative preferred embodiments, a PRKC-modulating agent binds a PRKC substrate, binding partner, or cofactor.

### Antibodies

In another embodiment, the protein modulator is a PRKC specific antibody agonist or antagonist. The antibodies have therapeutic and diagnostic utilities, and can be used in screening assays to identify PRKC modulators. The antibodies can also be used in dissecting the portions of the PRKC pathway responsible for various cellular responses and in the general processing and maturation of the PRKC.

Antibodies that specifically bind PRKC polypeptides can be generated using known methods. Preferably the antibody is specific to a mammalian ortholog of PRKC polypeptide, and more preferably, to human PRKC. Antibodies may be polyclonal, monoclonal (mAbs), humanized or chimeric antibodies, single chain antibodies, Fab fragments, F(ab').sub.2 fragments, fragments produced by a FAb expression library, anti-idiotypic (anti-Id) antibodies, and epitope-binding fragments of any of the above. Epitopes of PRKC which are particularly antigenic can be selected, for example, by routine screening of PRKC polypeptides for antigenicity or by applying a theoretical method for selecting antigenic regions of a protein (Hopp and Wood (1981), Proc. Nati. Acad. Sci. U.S.A. 78:3824-28; Hopp and Wood, (1983) Mol. Immunol. 20:483-89; Sutcliffe et al., (1983) Science 219:660-66) to the amino acid sequence of a PRKC. Monoclonal antibodies with affinities of 10⁸ M⁻¹ preferably 10⁹ M⁻¹ to 10¹⁰ M⁻¹, or stronger can be made by standard procedures as described (Harlow and Lane, *supra*; Goding (1986) Monoclonal Antibodies: Principles and Practice (2d ed) Academic Press, New York; and U.S. Pat. Nos. 4,381,292; 4,451,570; and 4,618,577). Antibodies may be generated against crude cell extracts of PRKC or substantially purified fragments thereof. If PRKC fragments are used, they preferably comprise at least 10, and more preferably, at least 20 contiguous amino acids of a PRKC protein. In a particular embodiment, PRKC-specific antigens and/or immunogens are coupled to carrier proteins that stimulate the immune response. For example, the subject polypeptides are covalently coupled to the keyhole limpet hemocyanin (KLH) carrier, and the conjugate is emulsified in Freund's complete adjuvant, which enhances the immune response. An appropriate immune system such as a laboratory rabbit or mouse is immunized according to conventional protocols.

The presence of PRKC-specific antibodies is assayed by an appropriate assay such as a solid phase enzyme-linked immunosorbant assay (ELISA) using immobilized corresponding PRKC polypeptides. Other assays, such as radioimmunoassays or fluorescent assays might also be used.

Chimeric antibodies specific to PRKC polypeptides can be made that contain different portions from different animal species. For instance, a human immunoglobulin constant region may be linked to a variable region of a murine mAb, such that the antibody derives its biological activity from the human antibody, and its binding specificity from the murine fragment. Chimeric antibodies are produced by splicing together genes that encode the appropriate regions from each species (Morrison et al., Proc. Natl. Acad. Sci. (1984) 81:6851-6855; Neuberger et al., Nature (1984) 312:604-608; Takeda et al., Nature (1985) 31:452-454). Humanized antibodies, which are a form of chimeric antibodies, can be generated by grafting complementary-determining regions (CDRs) (Carlos, T. M., J. M. Harlan. 1994. Blood 84:2068-2101) of mouse antibodies into a background of human framework regions and constant regions by recombinant DNA technology (Riechmann LM, et al., 1988 Nature 323: 323-327). Humanized antibodies contain ∼10% murine sequences and ∼90% human sequences, and thus further reduce or eliminate immunogenicity, while retaining the antibody specificities (Co MS, and Queen C. 1991 Nature 351: 501-501; Morrison SL. 1992 Ann. Rev. Immun. 10:239-265). Humanized antibodies and methods of their production are well-known in the art (U.S. Pat. Nos. 5,530,101, 5,585,089, 5,693,762, and 6,180,370).

PRKC-specific single chain antibodies which are recombinant, single chain polypeptides formed by linking the heavy and light chain fragments of the Fv regions via an amino acid bridge, can be produced by methods known in the art (U.S. Pat. No. 4,946,778; Bird, Science (1988) 242:423-426; Huston et al., Proc. Natl. Acad. Sci. USA (1988) 85:5879-5883; and Ward et al., Nature (1989) 334:544-546).

Other suitable techniques for antibody production involve in vitro exposure of lymphocytes to the antigenic polypeptides or alternatively to selection of libraries of antibodies in phage or similar vectors (Huse et al., Science (1989) 246:1275-1281). As used herein, T-cell antigen receptors are included within the scope of antibody modulators (Harlow and Lane, 1988, *supra).*

The polypeptides and antibodies of the present invention may be used with or without modification. Frequently, antibodies will be labeled by joining, either covalently or non-covalently, a substance that provides for a detectable signal, or that is toxic to cells that express the targeted protein (Menard S, et al., Int J. Biol Markers (1989) 4:131-134). A wide variety of labels and conjugation techniques are known and are reported extensively in both the scientific and patent literature. Suitable labels include radionuclides, enzymes, substrates, cofactors, inhibitors, fluorescent moieties, fluorescent emitting lanthanide metals, chemiluminescent moieties, bioluminescent moieties, magnetic particles, and the like (U.S. Pat. Nos. 3,817,837; 3,850,752; 3,939,350; 3,996,345; 4,277,437; 4,275,149; and 4,366,241). Also, recombinant immunoglobulins may be produced (U.S. Pat. No. 4,816,567). Antibodies to cytoplasmic polypeptides may be delivered and reach their targets by conjugation with membrane-penetrating toxin proteins (U.S. Pat. No. 6,086,900).

When used therapeutically in a patient, the antibodies of the subject invention can be typically administered parenterally, when possible at the target site, or intravenously. The therapeutically effective dose and dosage regimen is determined by clinical studies. Typically, the amount of antibody administered is in the range of about 0.1 mg/kg -to about 10 mg/kg of patient weight. For parenteral administration, the antibodies are formulated in a unit dosage injectable form (e.g., solution, suspension, emulsion) in association with a pharmaceutically acceptable vehicle. Such vehicles are inherently nontoxic and non-therapeutic. Examples are water, saline, Ringer's solution, dextrose solution, and 5% human serum albumin. Nonaqueous vehicles such as fixed oils, ethyl oleate, or liposome carriers may also be used. The vehicle may contain minor amounts of additives, such as buffers and preservatives, which enhance isotonicity and chemical stability or otherwise enhance therapeutic potential. The antibodies' concentrations in such vehicles are typically in the range of about 1 mg/ml to about10 mg/ml. Immunotherapeutic methods are further described in the literature (US Pat. No. 5,859,206; WO0073469).

### Nucleic Acid Modulators

Other preferred PRKC-modulating agents comprise nucleic acid molecules, such as antisense oligomers or double stranded RNA (dsRNA), which generally inhibit PRKC activity. Preferred nucleic acid modulators interfere with the function of the PRKC nucleic acid such as DNA replication, transcription, translocation of the PRKC RNA to the site of protein translation, translation of protein from the PRKC RNA, splicing of the PRKC RNA to yield one or more mRNA species, or catalytic activity which may be engaged in or facilitated by the PRKC RNA.

In one embodiment, the antisense oligomer is an oligonucleotide that is sufficiently complementary to a PRKC mRNA to bind to and prevent translation, preferably by binding to the 5' untranslated region. PRKC-specific antisense oligonucleotides, preferably range from at least 6 to about 200 nucleotides. In some embodiments the oligonucleotide is preferably at least 10, 15, or 20 nucleotides in length. In other embodiments, the oligonucleotide is preferably less than 50, 40, or 30 nucleotides in length. The oligonucleotide can be DNA or RNA or a chimeric mixture or derivatives or modified versions thereof, single-stranded or double-stranded. The oligonucleotide can be modified at the base moiety, sugar moiety, or phosphate backbone. The oligonucleotide may include other appending groups such as peptides, agents that facilitate transport across the cell membrane, hybridization-triggered cleavage agents, and intercalating agents.

In another embodiment, the antisense oligomer is a phosphothioate morpholino oligomer (PMO). PMOs are assembled from four different morpholino subunits, each of which contain one of four genetic bases (A, C, G, or T) linked to a six-membered morpholine ring. Polymers of these subunits are joined by non-ionic phosphodiamidate intersubunit linkages. Details of how to make and use PMOs and other antisense oligomers are well known in the art (e.g. see WO99/18193; Probst JC, Antisense Oligodeoxynucleotide and Ribozyme Design, Methods. (2000) 22(3):271-281; Summerton J, and Weller D. 1997 Antisense Nucleic Acid Drug Dev. :7:187-95; US Pat. No. 5,235,033; and US Pat No. 5,378,841).

Alternative preferred PRKC nucleic acid modulators are double-stranded RNA species mediating RNA interference (RNAi). RNAi is the process of sequence-specific, post-transcriptional gene silencing in animals and plants, initiated by double-stranded RNA (dsRNA) that is homologous in sequence to the silenced gene. Methods relating to the use of RNAi to silence genes in *C. elegans, Drosophila*, plants, and humans are known in the art (Fire A, et al., 1998 Nature 391:806-811; Fire, A. Trends Genet. 15, 358-363 (1999); Sharp, P. A. RNA interference 2001. Genes Dev. 15,485-490 (2001); Hammond, S. M., et al., Nature Rev. Genet. 2, 110-1119 (2001); Tuschl, T. Chem. Biochem. 2,239-245 (2001); Hamilton, A. et al., Science 286, 950-952 (1999); Hammond, S. M., et al., Nature 404, 293-296 (2000); Zamore, P. D., et al., Cell 101, 25-33 (2000); Bernstein, E., et al., Nature 409, 363-366 (2001); Elbashir, S. M., et al., Genes Dev. 15, 188-200 (2001); WO0129058; WO9932619; Elbashir SM, et al., 2001 Nature 411:494-498).

Nucleic acid modulators are commonly used as research reagents, diagnostics, and therapeutics. For example, antisense oligonucleotides, which are able to inhibit gene expression with exquisite specificity, are often used to elucidate the function of particular genes (see, for example, U.S. Pat. No. 6,165,790). Nucleic acid modulators are also used, for example, to distinguish between functions of various members of a biological pathway. For example, antisense oligomers have been employed as therapeutic moieties in the treatment of disease states in animals and man and have been demonstrated in numerous clinical trials to be safe and effective (Milligan JF, et al, Current Concepts in Antisense Drug Design, J Med Chem. (1993) 36:1923-1937; Tonkinson JL et al., Antisense Oligodeoxynucleotides as Clinical Therapeutic Agents, Cancer Invest. (1996) 14:54-65). Accordingly, in one aspect of the invention, a PRKC-specific nucleic acid modulator is used in an assay to further elucidate the role of the PRKC in the beta catenin pathway, and/or its relationship to other members of the pathway. In another aspect of the invention, a PRKC-specific antisense oligomer is used as a therapeutic agent in the treatment of beta catenin-related disease states.

### Assay Systems

The invention provides assay systems and screening methods for identifying specific modulators of PRKC activity. As used herein, an "assay system" encompasses all the components required for performing and analyzing results of an assay that detects and/or measures a particular event. In general, primary assays are used to identify or confirm a modulator's specific biochemical or molecular effect with respect to the PRKC nucleic acid or protein. In general, secondary assays further assess the activity of a PRKC modulating agent identified by a primary assay and may confirm that the modulating agent affects PRKC in a manner relevant to the beta catenin pathway. In some cases, PRKC modulators will be directly tested in a secondary assay.

In a preferred embodiment, the screening method comprises contacting a suitable assay system comprising a PRKC polypeptide or nucleic acid with a candidate agent under conditions whereby, but for the presence of the agent, the system provides a reference activity (e.g. kinase activity), which is based on the particular molecular event the screening method detects. A statistically significant difference between the agent-biased activity and the reference activity indicates that the candidate agent modulates PRKC activity, and hence the beta catenin pathway. The PRKC polypeptide or nucleic acid used in the assay may comprise any of the nucleic acids or polypeptides described above.

### Primary Assays

The type of modulator tested generally determines the type of primary assay.

### Primary assays for small molecule modulators

For small molecule modulators, screening assays are used to identify candidate modulators. Screening assays may be cell-based or may use a cell-free system that recreates or retains the relevant biochemical reaction of the target protein (reviewed in Sittampalam GS et al., Curr Opin Chem Biol (1997) 1:384-91 and accompanying references). As used herein the term "cell-based" refers to assays using live cells, dead cells, or a particular cellular fraction, such as a membrane, endoplasmic reticulum, or mitochondrial fraction. The term "cell free" encompasses assays using substantially purified protein (either endogenous or recombinantly produced), partially purified or crude cellular extracts. Screening assays may detect a variety of molecular events, including protein-DNA interactions, protein-protein interactions (e.g., receptor-ligand binding), transcriptional activity (e.g., using a reporter gene), enzymatic activity (*e*.*g*., via a property of the substrate), activity of second messengers, immunogenicty and changes in cellular morphology or other cellular characteristics. Appropriate screening assays may use a wide range of detection methods including fluorescent, radioactive, colorimetric, spectrophotometric, and amperometric methods, to provide a read-out for the particular molecular event detected.

Cell-based screening assays usually require systems for recombinant expression of PRKC and any auxiliary proteins demanded by the particular assay. Appropriate methods for generating recombinant proteins produce sufficient quantities of proteins that retain their relevant biological activities and are of sufficient purity to optimize activity and assure assay reproducibility. Yeast two-hybrid and variant screens, and mass spectrometry provide preferred methods for determining protein-protein interactions and elucidation of protein complexes. In certain applications, when PRKC-interacting proteins are used in screens to identify small molecule modulators, the binding specificity of the interacting protein to the PRKC protein may be assayed by various known methods such as substrate processing (e.g. ability of the candidate PRKC-specific binding agents to function as negative effectors in PRKC-expressing cells), binding equilibrium constants (usually at least about 10⁷ M⁻¹, preferably at least about 10⁸ M⁻¹, more preferably at least about 10⁹ M⁻¹), and immunogenicity (e.g. ability to elicit PRKC specific antibody in a heterologous host such as a mouse, rat, goat or rabbit). For enzymes and receptors, binding may be assayed by, respectively, substrate and ligand processing.

The screening assay may measure a candidate agent's ability to specifically bind to or modulate activity of a PRKC polypeptide, a fusion protein thereof, or to cells or membranes bearing the polypeptide or fusion protein. The PRKC polypeptide can be full length or a fragment thereof that retains functional PRKC activity. The PRKC polypeptide may be fused to another polypeptide, such as a peptide tag for detection or anchoring, or to another tag. The PRKC polypeptide is preferably human PRKC, or is an ortholog or derivative thereof as described above. In a preferred embodiment, the screening assay detects candidate agent-based modulation of PRKC interaction with a binding target, such as an endogenous or exogenous protein or other substrate that has PRKC -specific binding activity, and can be used to assess normal PRKC gene function.

Suitable assay formats that may be adapted to screen for PRKC modulators are known in the art. Preferred screening assays are high throughput or ultra high throughput and thus provide automated, cost-effective means of screening compound libraries for lead compounds (Fernandes PB, Curr Opin Chem Biol (1998) 2:597-603; Sundberg SA, Curr Opin Biotechnol 2000, 11:47-53). In one preferred embodiment, screening assays uses fluorescence technologies, including fluorescence polarization, time-resolved fluorescence, and fluorescence resonance energy transfer. These systems offer means to monitor protein-protein or DNA-protein interactions in which the intensity of the signal emitted from dye-labeled molecules depends upon their interactions with partner molecules (e.g., Selvin PR, Nat Struct Biol (2000) 7:730-4; Fernandes PB, *supra*; Hertzberg RP and Pope AJ, Curr Opin Chem Biol (2000) 4:445-451).

A variety of suitable assay systems may be used to identify candidate PRKC and beta catenin pathway modulators (e.g. U.S. Pat. No. 6,165,992 (kinase assays); U.S. Pat. Nos. 5,550,019 and 6,133,437 (apoptosis assays); and U.S. Pat. Nos. 5,976,782, 6,225,118 and 6,444,434 (angiogenesis assays), among others). Specific preferred assays are described in more detail below.

**Kinase assays.** In some preferred embodiments the screening assay detects the ability of the test agent to modulate the kinase activity of a PRKC polypeptide. In further embodiments, a cell-free kinase assay system is used to identify a candidate beta catenin modulating agent, and a secondary, cell-based assay, such as an apoptosis or hypoxic induction assay (described below), may be used to further characterize the candidate beta catenin modulating agent. Many different assays for kinases have been reported in the literature and are well known to those skilled in the art (e.g. U.S. Pat. No. 6,165,992; Zhu et al., Nature Genetics (2000) 26:283-289; and WO0073469). Radioassays, which monitor the transfer of a gamma phosphate are frequently used. For instance, a scintillation assay for p56 (lck) kinase activity monitors the transfer of the gamma phosphate from gamma -³³P ATP to a biotinylated peptide substrate; the substrate is captured on a streptavidin coated bead that transmits the signal (Beveridge M et al., J Biomol Screen (2000) 5:205-212). This assay uses the scintillation proximity assay (SPA), in which only radio-ligand bound to receptors tethered to the surface of an SPA bead are detected by the scintillant immobilized within it, allowing binding to be measured without separation of bound from free ligand.

Other assays for protein kinase activity may use antibodies that specifically recognize phosphorylated substrates. For instance, the kinase receptor activation (KIRA) assay measures receptor tyrosine kinase activity by ligand stimulating the intact receptor in cultured cells, then capturing solubilized receptor with specific antibodies and quantifying phosphorylation via phosphotyrosine ELISA (Sadick MD, Dev Biol Stand (1999) 97:121-133).

Another example of antibody based assays for protein kinase activity is TRF (time-resolved fluorometry). This method utilizes europium chelate-labeled anti-phosphotyrosine antibodies to detect phosphate transfer to a polymeric substrate coated onto microtiter plate wells. The amount of phosphorylation is then detected using time-resolved, dissociation-enhanced fluorescence (Braunwalder AF, et al., Anal Biochem 1996 Jul 1;238(2):159-64).

**Apoptosis assays.** Apoptosis or programmed cell death is a suicide program is activated within the cell, leading to fragmentation of DNA, shrinkage of the cytoplasm, membrane changes and cell death. Apoptosis is mediated by proteolytic enzymes of the caspase family. Assays for apoptosis may be performed by terminal deoxynucleotidyl transferase-mediated digoxigenin-11-dUTP nick end labeling (TUNEL) assay. The TUNEL assay is used to measure nuclear DNA fragmentation characteristic of apoptosis ( Lazebnik et al., 1994, Nature 371, 346), by following the incorporation of fluorescein-dUTP (Yonehara et al., 1989, J. Exp. Med. 169, 1747). Apoptosis may further be assayed by acridine orange staining of tissue culture cells (Lucas, R., et al., 1998, Blood 15:4730-41). Other cell-based apoptosis assays include the caspase-3/7 assay and the cell death nucleosome ELISA assay. The caspase 3/7 assay is based on the activation of the caspase cleavage activity as part of a cascade of events that occur during programmed cell death in many apoptotic pathways. In the caspase 3/7 assay (commercially available Apo-ONE^{™} Homogeneous Caspase-3/7 assay from Promega, cat# 67790), lysis buffer and caspase substrate are mixed and added to cells. The caspase substrate becomes fluorescent when cleaved by active caspase 3/7. The nucleosome ELISA assay is a general cell death assay known to those skilled in the art, and available commercially (Roche, Cat# 1774425). This assay is a quantitative sandwich-enzyme-immunoassay which uses monoclonal antibodies directed against DNA and histones respectively, thus specifically determining amount of mono- and oligonucleosomes in the cytoplasmic fraction of cell lysates. Mono and oligonucleosomes are enriched in the cytoplasm during apoptosis due to the fact that DNA fragmentation occurs several hours before the plasma membrane breaks down, allowing for accumalation in the cytoplasm. Nucleosomes are not present in the cytoplasmic fraction of cells that are not undergoing apoptosis. The Phospho-histone H2B assay is another apoptosis assay, based on phosphorylation of histone H2B as a result of apoptosis. Fluorescent dyes that are associated with phosphohistone H2B may be used to measure the increase of phosphohistone H2B as a result of apoptosis. Apoptosis assays that simultaneously measure multiple parameters associated with apoptosis have also been developed. In such assays, various cellular parameters that can be associated with antibodies or fluorescent dyes, and that mark various stages of apoptosis are labeled, and the results are measured using instruments such as Cellomics™ ArrayScan^{®} HCS System. The measurable parameters and their markers include anti-active caspase-3 antibody which marks intermediate stage apoptosis, anti-PARP-p85 antibody (cleaved PARP) which marks late stage apoptosis, Hoechst labels which label the nucleus and are used to measure nuclear swelling as a measure of early apoptosis and nuclear condensation as a measure of late apoptosis, and TOTO-3 fluorescent dye which labels DNA of dead cells with high cell membrane permeability.

An apoptosis assay system may comprise a cell that expresses a PRKC, and that optionally has defective beta catenin function (e.g. beta catenin is over-expressed or under-expressed relative to wild-type cells). A test agent can be added to the apoptosis assay system and changes in induction of apoptosis relative to controls where no test agent is added, identify candidate beta catenin modulating agents. In some embodiments of the invention, an apoptosis assay may be used as a secondary assay to test a candidate beta catenin modulating agents that is initially identified using a cell-free assay system. An apoptosis assay may also be used to test whether PRKC function plays a direct role in apoptosis. For example, an apoptosis assay may be performed on cells that over- or under-express PRKC relative to wild type cells. Differences in apoptotic response compared to wild type cells suggests that the PRKC plays a direct role in the apoptotic response. Apoptosis assays are described further in US Pat. No. 6,133,437.

**Cell proliferation and cell cycle assays.** Cell proliferation may be assayed via bromodeoxyuridine (BRDU) incorporation. This assay identifies a cell population undergoing DNA synthesis by incorporation of BRDU into newly-synthesized DNA. Newly-synthesized DNA may then be detected using an anti-BRDU antibody (Hoshino et al., 1986, Int. J. Cancer 38, 369; Campana et al., 1988, J. Immunol. Meth. 107, 79), or by other means.

Cell proliferation is also assayed via phospho-histone H3 staining, which identifies a cell population undergoing mitosis by phosphorylation of histone H3. Phosphorylation of histone H3 at serine 10 is detected using an antibody specfic to the phosphorylated form of the serine 10 residue of histone H3. (Chadlee,D.N. 1995, J. Biol. Chem 270:20098-105). Cell Proliferation may also be examined using [³H]-thymidine incorporation (Chen, J., 1996, Oncogene 13:1395-403; Jeoung, J., 1995, J. Biol. Chem. 270:18367-73). This assay allows for quantitative characterization of S-phase DNA syntheses. In this assay, cells synthesizing DNA will incorporate [³H]-thymidine into newly synthesized DNA. Incorporation can then be measured by standard techniques such as by counting of radioisotope in a scintillation counter (e.g., Beckman LS 3800 Liquid Scintillation Counter). Another proliferation assay uses the dye Alamar Blue (available from Biosource International), which fluoresces when reduced in living cells and provides an indirect measurement of cell number (Voytik-Harbin SL et al., 1998, In Vitro Cell Dev Biol Anim 34:239-46). Yet another proliferation assay, the MTS assay, is based on in vitro cytotoxicity assessment of industrial chemicals, and uses the soluble tetrazolium salt, MTS. MTS assays are commercially available, for example, the Promega CellTiter 96^{®} AQueous Non-Radioactive Cell Proliferation Assay (Cat.# G5421).

Cell proliferation may also be assayed by colony formation in soft agar, or clonogenic survival assay (Sambrook et al., Molecular Cloning, Cold Spring Harbor (1989)). For example, cells transformed with PRKC are seeded in soft agar plates, and colonies are measured and counted after two weeks incubation.

Cell proliferation may also be assayed by measuring ATP levels as indicator of metabolically active cells. Such assays are commercially available, for example Cell Titer-Glo™, which is a luminescent homogeneous assay available from Promega.

Involvement of a gene in the cell cycle may be assayed by flow cytometry (Gray JW et al. (1986) Int J Radiat Biol Relat Stud Phys Chem Med 49:237-55). Cells transfected with a PRKC may be stained with propidium iodide and evaluated in a flow cytometer (available from Becton Dickinson), which indicates accumulation of cells in different stages of the cell cycle.

Accordingly, a cell proliferation or cell cycle assay system may comprise a cell that expresses a PRKC, and that optionally has defective beta catenin function (e.g. beta catenin is over-expressed or under-expressed relative to wild-type cells). A test agent can be added to the assay system and changes in cell proliferation or cell cycle relative to controls where no test agent is added, identify candidate beta catenin modulating agents. In some embodiments of the invention, the cell proliferation or cell cycle assay may be used as a secondary assay to test a candidate beta catenin modulating agents that is initially identified using another assay system such as a cell-free assay system. A cell proliferation assay may also be used to test whether PRKC function plays a direct role in cell proliferation or cell cycle. For example, a cell proliferation or cell cycle assay may be performed on cells that over- or under-express PRKC relative to wild type cells. Differences in proliferation or cell cycle compared to wild type cells suggests that the PRKC plays a direct role in cell proliferation or cell cycle.

**Angiogenesis.** Angiogenesis may be assayed using various human endothelial cell systems, such as umbilical vein, coronary artery, or dermal cells. Suitable assays include Alamar Blue based assays (available from Biosource International) to measure proliferation; migration assays using fluorescent molecules, such as the use of Becton Dickinson Falcon HTS FluoroBlock cell culture inserts to measure migration of cells through membranes in presence or absence of angiogenesis enhancer or suppressors; and tubule formation assays based on the formation of tubular structures by endothelial cells on Matrigel® (Becton Dickinson). Accordingly, an angiogenesis assay system may comprise a cell that expresses a PRKC, and that optionally has defective beta catenin function (e.g. beta catenin is over-expressed or under-expressed relative to wild-type cells). A test agent can be added to the angiogenesis assay system and changes in angiogenesis relative to controls where no test agent is added, identify candidate beta catenin modulating agents. In some embodiments of the invention, the angiogenesis assay may be used as a secondary assay to test a candidate beta catenin modulating agents that is initially identified using another assay system. An angiogenesis assay may also be used to test whether PRKC function plays a direct role in cell proliferation. For example, an angiogenesis assay may be performed on cells that over- or under-express PRKC relative to wild type cells. Differences in angiogenesis compared to wild type cells suggests that the PRKC plays a direct role in angiogenesis. U.S. Pat. Nos. 5,976,782, 6,225,118 and 6,444,434, among others, describe various angiogenesis assays.

**Hypoxic induction**. The alpha subunit of the transcription factor, hypoxia inducible factor-1 (HIF-1), is upregulated in tumor cells following exposure to hypoxia in vitro. Under hypoxic conditions, HIF-1 stimulates the expression of genes known to be important in tumour cell survival, such as those encoding glyolytic enzymes and VEGF. Induction of such genes by hypoxic conditions may be assayed by growing cells transfected with PRKC in hypoxic conditions (such as with 0.1% 02, 5% CO2, and balance N2, generated in a Napco 7001 incubator (Precision Scientific)) and normoxic conditions, followed by assessment of gene activity or expression by Taqman®. For example, a hypoxic induction assay system may comprise a cell that expresses a PRKC, and that optionally has defective beta catenin function (e.g. beta catenin is over-expressed or under-expressed relative to wild-type cells). A test agent can be added to the hypoxic induction assay system and changes in hypoxic response relative to controls where no test agent is added, identify candidate beta catenin modulating agents. In some embodiments of the invention, the hypoxic induction assay may be used as a secondary assay to test a candidate beta catenin modulating agents that is initially identified using another assay system. A hypoxic induction assay may also be used to test whether PRKC function plays a direct role in the hypoxic response. For example, a hypoxic induction assay may be performed on cells that over- or under-express PRKC relative to wild type cells. Differences in hypoxic response compared to wild type cells suggests that the PRKC plays a direct role in hypoxic induction.

**Cell adhesion.** Cell adhesion assays measure adhesion of cells to purified adhesion proteins, or adhesion of cells to each other, in presence or absence of candidate modulating agents. Cell-protein adhesion assays measure the ability of agents to modulate the adhesion of cells to purified proteins. For example, recombinant proteins are produced, diluted to 2.5g/mL in PBS, and used to coat the wells of a microtiter plate. The wells used for negative control are not coated. Coated wells are then washed, blocked with 1% BSA, and washed again. Compounds are diluted to 2x final test concentration and added to the blocked, coated wells. Cells are then added to the wells, and the unbound cells are washed off. Retained cells are labeled directly on the plate by adding a membrane-permeable fluorescent dye, such as calcein-AM, and the signal is quantified in a fluorescent microplate reader.

Cell-cell adhesion assays measure the ability of agents to modulate binding of cell adhesion proteins with their native ligands. These assays use cells that naturally or recombinantly express the adhesion protein of choice. In an exemplary assay, cells expressing the cell adhesion protein are plated in wells of a multiwell plate. Cells expressing the ligand are labeled with a membrane-permeable fluorescent dye, such as BCECF , and allowed to adhere to the monolayers in the presence of candidate agents. Unbound cells are washed off, and bound cells are detected using a fluorescence plate reader.

High-throughput cell adhesion assays have also been described. In one such assay, small molecule ligands and peptides are bound to the surface of microscope slides using a microarray spotter, intact cells are then contacted with the slides, and unbound cells are washed off. In this assay, not only the binding specificity of the peptides and modulators against cell lines are determined, but also the functional cell signaling of attached cells using immunofluorescence techniques in situ on the microchip is measured (Falsey JR et al., Bioconjug Chem. 2001 May-Jun;12(3):346-53).

**Tubulogenesis.** Tubulogenesis assays monitor the ability of cultured cells, generally endothelial cells, to form tubular structures on a matrix substrate, which generally simulates the environment of the extracellular matrix. Exemplary substrates include Matrigel^{™} (Becton Dickinson), an extract of basement membrane proteins containing laminin, collagen IV, and heparin sulfate proteoglycan, which is liquid at 4° C and forms a solid gel at 37° C. Other suitable matrices comprise extracellular components such as collagen, fibronectin, and/or fibrin. Cells are stimulated with a pro-angiogenic stimulant, and their ability to form tubules is detected by imaging. Tubules can generally be detected after an overnight incubation with stimuli, but longer or shorter time frames may also be used. Tube formation assays are well known in the art (e.g., Jones MK et al., 1999, Nature Medicine 5:1418-1423). These assays have traditionally involved stimulation with serum or with the growth factors FGF or VEGF. Serum represents an undefined source of growth factors. In a preferred embodiment, the assay is performed with cells cultured in serum free medium, in order to control which process or pathway a candidate agent modulates. Moreover, we have found that different target genes respond differently to stimulation with different pro-angiogenic agents, including inflammatory angiogenic factors such as TNF-alpa. Thus, in a further preferred embodiment, a tubulogenesis assay system comprises testing a PRKC's response to a variety of factors, such as FGF, VEGF, phorbol myristate acetate (PMA), TNF-alpha, ephrin, etc.

**Cell Migration.** An invasion/migration assay (also called a migration assay) tests the ability of cells to overcome a physical barrier and to migrate towards pro-angiogenic signals. Migration assays are known in the art (e.g., Paik JH et al., 2001, J Biol Chem 276:11830-11837). In a typical experimental set-up, cultured endothelial cells are seeded onto a matrix-coated porous lamina, with pore sizes generally smaller than typical cell size. The matrix generally simulates the environment of the extracellular matrix, as described above. The lamina is typically a membrane, such as the transwell polycarbonate membrane (Coming Costar Corporation, Cambridge, MA), and is generally part of an upper chamber that is in fluid contact with a lower chamber containing pro-angiogenic stimuli. Migration is generally assayed after an overnight incubation with stimuli, but longer or shorter time frames may also be used. Migration is assessed as the number of cells that crossed the lamina, and may be detected by staining cells with hemotoxylin solution (VWR Scientific, South San Francisco, CA), or by any other method for determining cell number. In another exemplary set up, cells are fluorescently labeled and migration is detected using fluorescent readings, for instance using the Falcon HTS FluoroBlok (Becton Dickinson). While some migration is observed in the absence of stimulus, migration is greatly increased in response to pro-angiogenic factors. As described above, a preferred assay system for migration/invasion assays comprises testing a PRKC's response to a variety of pro-angiogenic factors, including tumor angiogenic and inflammatory angiogenic agents, and culturing the cells in serum free medium.

**Sprouting assay**. A sprouting assay is a three-dimensional *in vitro* angiogenesis assay that uses a cell-number defined spheroid aggregation of endothelial cells ("spheroid"), embedded in a collagen gel-based matrix. The spheroid can serve as a starting point for the sprouting of capillary-like structures by invasion into the extracellular matrix (termed "cell sprouting") and the subsequent formation of complex anastomosing networks (Korff and Augustin, 1999, J Cell Sci 112:3249-58). In an exemplary experimental set-up, spheroids are prepared by pipetting 400 human umbilical vein endothelial cells into individual wells of a nonadhesive 96-well plates to allow overnight spheroidal aggregation (Korff and Augustin: J Cell Biol 143: 1341-52, 1998). Spheroids are harvested and seeded in 900µl of methocel-collagen solution and pipetted into individual wells of a 24 well plate to allow collagen gel polymerization. Test agents are added after 30 min by pipetting 100 µl of 10-fold concentrated working dilution of the test substances on top of the gel. Plates are incubated at 37°C for 24h. Dishes are fixed at the end of the experimental incubation period by addition of paraformaldehyde. Sprouting intensity of endothelial cells can be quantitated by an automated image analysis system to determine the cumulative sprout length per spheroid.

### Primary assays for antibody modulators

For antibody modulators, appropriate primary assays test is a binding assay that tests the antibody's affinity to and specificity for the PRKC protein. Methods for testing antibody affinity and specificity are well known in the art (Harlow and Lane, 1988, 1999, *supra).* The enzyme-linked immunosorbant assay (ELISA) is a preferred method for detecting PRKC-specific antibodies; others include FACS assays, radioimmunoassays, and fluorescent assays.

In some cases, screening assays described for small molecule modulators may also be used to test antibody modulators.

### Primary assays for nucleic acid modulators

For nucleic acid modulators, primary assays may test the ability of the nucleic acid modulator to inhibit or enhance PRKC gene expression, preferably mRNA expression. In general, expression analysis comprises comparing PRKC expression in like populations of cells (*e*.*g*., two pools of cells that endogenously or recombinantly express PRKC) in the presence and absence of the nucleic acid modulator. Methods for analyzing mRNA and protein expression are well known in the art. For instance, Northern blotting, slot blotting, ribonuclease protection, quantitative RT-PCR (e.g., using the TaqMan®, PE Applied Biosystems), or microarray analysis may be used to confirm that PRKC mRNA expression is reduced in cells treated with the nucleic acid modulator (e.g., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm DH and Guiseppi-Elie, A Curr Opin Biotechnol 2001, 12:41-47). Protein expression may also be monitored. Proteins are most commonly detected with specific antibodies or antisera directed against either the PRKC protein or specific peptides. A variety of means including Western blotting, ELISA, or in situ detection, are available (Harlow E and Lane D, 1988 and 1999, *supra).*

In some cases, screening assays described for small molecule modulators, particularly in assay systems that involve PRKC mRNA expression, may also be used to test nucleic acid modulators.

### Secondary Assays

Secondary assays may be used to further assess the activity of PRKC-modulating agent identified by any of the above methods to confirm that the modulating agent affects PRKC in a manner relevant to the beta catenin pathway. As used herein, PRKC-modulating agents encompass candidate clinical compounds or other agents derived from previously identified modulating agent. Secondary assays can also be used to test the activity of a modulating agent on a particular genetic or biochemical pathway or to test the specificity of the modulating agent's interaction with PRKC.

Secondary assays generally compare like populations of cells or animals (e.g., two pools of cells or animals that endogenously or recombinantly express PRKC) in the presence and absence of the candidate modulator. In general, such assays test whether treatment of cells or animals with a candidate PRKC-modulating agent results in changes in the beta catenin pathway in comparison to untreated (or mock- or placebo-treated) cells or animals. Certain assays use "sensitized genetic backgrounds", which, as used herein, describe cells or animals engineered for altered expression of genes in the beta catenin or interacting pathways.

### Cell-based assays

Cell based assays may detect endogenous beta catenin pathway activity or may rely on recombinant expression of beta catenin pathway components. Any of the aforementioned assays may be used in this cell-based format. Candidate modulators are typically added to the cell media but may also be injected into cells or delivered by any other efficacious means.

### Animal Assays

A variety of non-human animal models of normal or defective beta catenin pathway are described herein. Models for defective beta catenin pathway typically use genetically modified animals that have been engineered to mis-express (e.g., over-express or lack expression in) genes involved in the beta catenin pathway. Assays generally require systemic delivery of the candidate modulators, such as by oral administration, injection, etc.

Beta catenin pathway activity can be assessed by monitoring neovascularization and angiogenesis. Animal models with defective and normal beta catenin can be used to test the candidate modulator's affect on PRKC in Matrigel® assays. Matrigel® is an extract of basement membrane proteins, and is composed primarily of laminin, collagen IV, and heparin sulfate proteoglycan. It is provided as a sterile liquid at 4° C, but rapidly forms a solid gel at 37° C. Liquid Matrigel® is mixed with various angiogenic agents, such as bFGF and VEGF, or with human tumor cells which over-express the PRKC. The mixture is then injected subcutaneously(SC) into female athymic nude mice (Taconic, Germantown, NY) to support an intense vascular response. Mice with Matrlgel® pellets may be dosed via oral (PO), intraperitoneal (IP), or intravenous (IV) routes with the candidate modulator. Mice are euthanized 5 - 12 days post-injection, and the Matrigel® pellet is harvested for hemoglobin analysis (Sigma plasma hemoglobin kit). Hemoglobin content of the gel is found to correlate the degree of neovascularization in the gel.

The effect of the candidate modulator on PRKC can also be assessed via tumorigenicity assays. Tumor xenograft assays are known in the art (see, e.g., Ogawa K et al., 2000, Oncogene 19:6043-6052). Xenografts are typically implanted SC into female athymic mice, 6-7 week old, as single cell suspensions either from a pre-existing tumor or from *in vitro* culture. The tumors which express the PRKC endogenously are injected in the flank, 1 x 10⁵ to 1 x 10⁷ cells per mouse in a volume of 100 µL using a 27gauge needle. Mice are then ear tagged and tumors are measured twice weekly. Candidate modulator treatment is initiated on the day the mean tumor weight reaches 100 mg. Candidate modulator is delivered IV, SC, IP, or PO by bolus administration. Depending upon the pharmacokinetics of each unique candidate modulator, dosing can be performed multiple times per day. The tumor weight is assessed by measuring perpendicular diameters with a caliper and calculated by multiplying the measurements of diameters in two dimensions. At the end of the experiment, the excised tumors maybe utilized for biomarker identification or further analyses. For immunohistochemistry staining, xenograft tumors are fixed in 4% paraformaldehyde, 0.1M phosphate, pH 7.2, for 6 hours at 4°C, immersed in 30% sucrose in PBS, and rapidly frozen in isopentane cooled with liquid nitrogen.

Tumorogenicity can also be monitored using a hollow fiber assay, which is described in U.S. Pat No. US 5,698,413. Briefly, the method comprises implanting into a laboratory animal a biocompatible, semi-permeable encapsulation device containing target cells, treating the laboratory animal with a candidate modulating agent, and evaluating the target cells for reaction to the candidate modulator. Implanted cells are generally human cells from a pre-existing tumor or a tumor cell line. After an appropriate period of time, generally around six days, the implanted samples are harvested for evaluation of the candidate modulator. Tumorogenicity and modulator efficacy may be evaluated by assaying the quantity of viable cells present in the macrocapsule, which can be determined by tests known in the art, for example, MTT dye conversion assay, neutral red dye uptake, trypan blue staining, viable cell counts, the number of colonies formed in soft agar, the capacity of the cells to recover and replicate in vitro, etc.

Tumorogenicity assays can use a transgenic animal, usually a mouse, carrying a dominant oncogene or tumor suppressor gene knockout under the control of tissue specific regulatory sequences; these assays are generally referred to as transgenic tumor assays. In a preferred application, tumor development in the transgenic model is well characterized or is controlled. In an exemplary model, the "RIP1-Tag2" transgene, comprising the SV40 large T-antigen oncogene under control of the insulin gene regulatory regions is expressed in pancreatic beta cells and results in islet cell carcinomas (Hanahan D, 1985, Nature 315:115-122; Parangi S et al, 1996, Proc Natl Acad Sci USA 93: 2002-2007; Bergers G et al, 1999, Science 284:808-812). An "angiogenic switch," occurs at approximately five weeks, as normally quiescent capillaries in a subset of hyperproliferative islets become angiogenic. The RIP1-TAG2 mice die by age 14 weeks. Candidate modulators may be administered at a variety of stages, including just prior to the angiogenic switch (e.g., for a model of tumor prevention), during the growth of small tumors (e.g., for a model of intervention), or during the growth of large and/or invasive tumors (e.g., for a model of regression). Tumorogenicity and modulator efficacy can be evaluating life-span extension and/or tumor characteristics, including number of tumors, tumor size, tumor morphology, vessel density, apoptotic index, etc.

### Diagnostic and therapeutic uses

Specific PRKC-modulating agents are useful in a variety of diagnostic and therapeutic applications where disease or disease prognosis is related to defects in the beta catenin pathway, such as angiogenic, apoptotic, or cell proliferation disorders. Accordingly, the invention also provides in vitro methods for modulating the beta catenin pathway in a cell, preferably a cell pre-determined to have defective or impaired beta catenin function (e.g. due to overexpression, underexpression, or misexpression of beta catenin, or due to gene mutations), comprising the step of contacting an agent to the cell that specifically modulates PRKC activity. Preferably, the modulating agent produces a detectable phenotypic change in the cell indicating that the beta catenin function is restored. The phrase "function is restored", and equivalents, as used herein, means that the desired phenotype is achieved, or is brought closer to normal compared to untreated cells. For example, with restored beta catenin function, cell proliferation and/or progression through cell cycle may normalize, or be brought closer to normal relative to untreated cells. The invention also provides agents for use in the treatment of disorders or disease associated with impaired beta catenin function. The invention further provides in vitro methods for modulating PRKC function in a cell, preferably a cell pre-determined to have defective or impaired PRKC function, by administering a PRKC -modulating agent.

The discovery that PRKC is implicated in beta catenin pathway provides for a variety of methods that can be employed for the diagnostic and prognostic evaluation of diseases and disorders involving defects in the beta catenin pathway and for the identification of subjects having a predisposition to such diseases and disorders.

Various expression analysis methods can be used to diagnose whether PRKC expression occurs in a particular sample, including Northern blotting, slot blotting, ribonuclease protection, quantitative RT-PCR, and microarray analysis. (e.g., Current Protocols in Molecular Biology (1994) Ausubel FM et al., eds., John Wiley & Sons, Inc., chapter 4; Freeman WM et al., Biotechniques (1999) 26:112-125; Kallioniemi OP, Ann Med 2001, 33:142-147; Blohm and Guiseppi-Elie, Curr Opin Biotechnol 2001, 12:41-47). Tissues having a disease or disorder implicating defective beta catenin signaling that express a PRKC, are identified as amenable to treatment with a PRKC modulating agent. In a preferred application, the beta catenin defective tissue overexpresses a PRKC relative to normal tissue. For example, a Northern blot analysis of mRNA from tumor and normal cell lines, or from tumor and matching normal tissue samples from the same patient, using full or partial PRKC cDNA sequences as probes, can determine whether particular tumors express or overexpress PRKC. Alternatively, the TaqMan® is used for quantitative RT-PCR analysis of PRKC expression in cell lines, normal tissues and tumor samples (PE Applied Biosystems).

Various other diagnostic methods may be performed, for example, utilizing reagents such as the PRKC oligonucleotides, and antibodies directed against a PRKC, as described above for: (1) the detection of the presence of PRKC gene mutations, or the detection of either over- or under-expression of PRKC mRNA relative to the non-disorder state; (2) the detection of either an over- or an under-abundance of PRKC gene product relative to the non-disorder state; and (3) the detection of perturbations or abnormalities in the signal transduction pathway mediated by PRKC.

Kits for detecting expression of PRKC in various samples, comprising at least one antibody specific to PRKC, all reagents and/or devices suitable for the detection of antibodies, the immobilization of antibodies, and the like, and instructions for using such kits in diagnosis or therapy are also disclosed.

Thus, in a specific embodiment, the invention is drawn to an in vitro method for diagnosing a disease or disorder in a patient that is associated with alterations in PRKC expression, the method comprising: a) contacting a biological sample from the patient with a probe for PRKC expression; b) comparing results from step (a) with a control; and c) determining whether step (b) indicates a likelihood of the disease or disorder, wherein the disease or disorder is selected from as angiogenic, apoptotic and cell proliferation disease or disorder. Preferably, the disease is cancer, most preferably a cancer as shown in TABLE 1. The probe may be either DNA with a sequence as shown in SEQ ID NOs: 1,2,4 or 5 or protein encoded by said nucleic acid sequence.

### EXAMPLES

### I. Drosophila beta catenin screen

Two dominant loss of function screens were carried out in *Drosophila* to identify genes that interact with the Wg cell signaling molecule, beta -catenin (Riggleman et al. (1990) Cell 63:549-560; Peifer et al. (1991) Development 111:1029-1043). Late stage activation of the pathway in the developing *Drosophila* eye leads to apoptosis (Freeman and Bienz (2001) EMBO reports 2: 157-162), whereas early stage activation leads to an overgrowth phenotype. We discovered that ectopic expression of the activated protein in the wing results in changes of cell fate into ectopic bristles and wing veins.

Each transgene was carried in a separate fly stock:
Stocks and genotypes were as follows:
   eye overgrowth transgene: isow; P{3.5 eyeless-Gal4}; P{arm(S56F)-pExp-UAS)}/TM6b;
   eye apoptosis transgene: y w; P{arm(S56F)-pExp-GMR}/CyO; and
   wing transgene: P{arm(ΔN)-pExp-VgMQ}/FM7c

In the first dominant loss of function screen, females of each of these three transgenes were crossed to a collection of males containing genomic deficiencies. Resulting progeny containing the transgene and the deficiency were then scored for the effect of the deficiency on the eye apoptosis, eye overgrowth, and wing phenotypes, i.e., whether the deficiency enhanced, suppressed, or had no effect on their respective phenotypes. All data was recorded and all modifiers were retested with a repeat of the original cross. Modifying deficiencies of the phenotypes were then prioritized according to how they modified each of the three phenotypes.

Transposons contained within the prioritized deficiencies were then screened as described. Females of each of the three transgenes were crossed to a collection of 4 types of transposons (3 piggyBac-based and 1 P-element-based). The resulting progeny containing the transgene and the transposon were scored for the effect of the transposon on their respective phenotypes. All data was recorded and all modifiers were retested with a repeat of the original cross. Modifiers of the phenotypes were identified as either members of the Wg pathway, components of apoptotic related pathways, components of cell cycle related pathways, or cell adhesion related proteins.

In the second dominant loss of function screen, females of the eye overgrowth transgene were crossed to males from a collection of 3 types of piggyBac-based transposons. The resulting progeny containing the transgene and the transposon were scored for the effect of the transposon on the eye overgrowth phenotype. All data was recorded and all modifiers were retested with a repeat of the original cross. Modifiers of the phenotypes were identified as either members of the Wg pathway, components of cell cycle related pathways, or cell adhesion related proteins.

BLAST analysis (Altschul et al., *supra*) was employed to identify orthologs of Drosophila modifiers. For example, representative sequences from PRKC, GI# 4506071 (SEQ ID NO:11), and GI#10864650 (SEQ ID NO:12) share 68% and 63% amino acid identity, respectively, with the *Drosophila a*PKC.

Various domains, signals, and functional subunits in proteins were analyzed using the PSORT (Nakai K., and Horton P., Trends Biochem Sci, 1999, 24:34-6; Kenta Nakai, Protein sorting signals and prediction of subcellular localization, Adv. Protein Chem. 54, 277-344 (2000)), PFAM (Bateman A., et al., Nucleic Acids Res, 1999, 27:260-2), SMART (Ponting CP, et al., SMART: identification and annotation of domains from signaling and extracellular protein sequences. Nucleic Acids Res. 1999 Jan 1;27(1):229-32), TM-HMM (Erik L.L. Sonnhammer, Gunnar von Heijne, and Anders Krogh: A hidden Markov model for predicting transmembrane helices in protein sequences. In Proc. of Sixth Int. Conf. on Intelligent Systems for Molecular Biology, p 175-182 Ed J. Glasgow, T. Littlejohn, F. Major, R. Lathrop, D. Sankoff, and C. Sensen Menlo Park, CA: AAAI Press, 1998), and clust (Remm M, and Sonnhammer E. Classification of transmembrane protein families in the Caenorhabditis elegans genome and identification of human orthologs. Genome Res. 2000 Nov;10(11): 1679-89) programs. For example, the kinase domain (PFAM 00069) of PRKC from GI#s 4506071 and 10864650 (SEQ ID NOs:11 and 12, respectively) is located respectively at approximately amino acid residues 245 to 513 and 252 to 518. Further, the Kinase C terminal domain (PFAM 00433) of the same proteins is located respectively at approximately amino acid residues 514 to 580 and 519 to 585.

### II. High-Throughput In Vitro Fluorescence Polarization Assay

Fluorescently-labeled PRKC peptide/substrate are added to each well of a 96-well microtiter plate, along with a test agent in a test buffer (10 mM HEPES, 10 mM NaCl, 6 mM magnesium chloride, pH 7.6). Changes in fluorescence polarization, determined by using a Fluorolite FPM-2 Fluorescence Polarization Microtiter System (Dynatech Laboratories, Inc), relative to control values indicates the test compound is a candidate modifier of PRKC activity.

### III. High-Throughput In Vitro Binding Assay.

³³P-labeled PRKC peptide is added in an assay buffer (100 mM KCl, 20 mM HEPES pH 7.6, 1 mM MgCl₂, 1% glycerol, 0.5% NP-40, 50 mM beta-mercaptoethanol, 1 mg/ml BSA, cocktail of protease inhibitors) along with a test agent to the wells of a Neutralite-avidin coated assay plate and incubated at 25°C for 1 hour. Biotinylated substrate is then added to each well and incubated for 1 hour. Reactions are stopped by washing with PBS, and counted in a scintillation counter. Test agents that cause a difference in activity relative to control without test agent are identified as candidate beta catenin modulating agents.

### IV. Immunoprecipitations and Immunoblotting

For coprecipitation of transfected proteins, 3 × 10⁶ appropriate recombinant cells containing the PRKC proteins are plated on 10-cm dishes and transfected on the following day with expression constructs. The total amount of DNA is kept constant in each transfection by adding empty vector. After 24 h, cells are collected, washed once with phosphate-buffered saline and lysed for 20 min on ice in 1 ml of lysis buffer containing 50 mM Hepes, pH 7.9, 250 mM NaCl, 20 mM -glycerophosphate, 1 mM sodium orthovanadate, 5 mM p-nitrophenyl phosphate, 2 mM dithiothreitol, protease inhibitors (complete, Roche Molecular Biochemicals), and 1% Nonidet P-40. Cellular debris is removed by centrifugation twice at 15,000 × g for 15 min. The cell lysate is incubated with 25 µl of M2 beads (Sigma) for 2 h at 4 °C with gentle rocking.

After extensive washing with lysis buffer, proteins bound to the beads are solubilized by boiling in SDS sample buffer, fractionated by SDS-polyacrylamide gel electrophoresis, transferred to polyvinylidene difluoride membrane and blotted with the indicated antibodies. The reactive bands are visualized with horseradish peroxidase coupled to the appropriate secondary antibodies and the enhanced chemiluminescence (ECL) Western blotting detection system (Amersham Pharmacia Biotech).

### V. Kinase assay

A purified or partially purified PRKC is diluted in a suitable reaction buffer, e.g., 50 mM Hepes, pH 7.5, containing magnesium chloride or manganese chloride (1-20 mM) and a peptide or polypeptide substrate, such as myelin basic protein or casein (1-10 µg/ml). The final concentration of the kinase is 1-20 nM. The enzyme reaction is conducted in microtiter plates to facilitate optimization of reaction conditions by increasing assay throughput. A 96-well microtiter plate is employed using a final volume 30-100 µl. The reaction is initiated by the addition of ³³P-gamma-ATP (0.5 µCi/ml) and incubated for 0.5 to 3 hours at room temperature. Negative controls are provided by the addition of EDTA, which chelates the divalent cation (Mg2⁺ or Mn²⁺) required for enzymatic activity. Following the incubation, the enzyme reaction is quenched using EDTA. Samples of the reaction are transferred to a 96-well glass fiber filter plate (MultiScreen, Millipore). The filters are subsequently washed with phosphate-buffered saline, dilute phosphoric acid (0.5%) or other suitable medium to remove excess radiolabeled ATP. Scintillation cocktail is added to the filter plate and the incorporated radioactivity is quantitated by scintillation counting (Wallac/Perkin Elmer). Activity is defined by the amount of radioactivity detected following subtraction of the negative control reaction value (EDTA quench).

### VI. Expression analysis

All cell lines used in the following experiments are NCI (National Cancer Institute) lines, and are available from ATCC (American Type Culture Collection, Manassas, VA 20110-2209). Normal and tumor tissues were obtained from Impath, UC Davis, Clontech, Stratagene, Ardais, Genome Collaborative, and Ambion.

TaqMan^{®} analysis was used to assess expression levels of the disclosed genes in various samples.

RNA was extracted from each tissue sample using Qiagen (Valencia, CA) RNeasy kits, following manufacturer's protocols, to a final concentration of 50ng/µl. Single stranded cDNA was then synthesized by reverse transcribing the RNA samples using random hexamers and 500ng of total RNA per reaction, following protocol 4304965 of Applied Biosystems (Foster City, CA).

Primers for expression analysis using TaqMan® assay (Applied Biosystems, Foster City, CA) were prepared according to the TaqMan® protocols, and the following criteria: a) primer pairs were designed to span introns to eliminate genomic contamination, and b) each primer pair produced only one product. Expression analysis was performed using a 7900HT instrument.

TaqMan® reactions were carried out following manufacturer's protocols, in 25 µl total volume for 96-well plates and 10 µl total volume for 384-well plates, using 300nM primer and 250 nM probe, and approximately 25ng of cDNA. The standard curve for result analysis was prepared using a universal pool of human cDNA samples, which is a mixture of cDNAs from a wide variety of tissues so that the chance that a target will be present in appreciable amounts is good. The raw data were normalized using 18S rRNA (universally expressed in all tissues and cells).

For each expression analysis, tumor tissue samples were compared with matched normal tissues from the same patient. A gene was considered overexpressed in a tumor when the level of expression of the gene was 2 fold or higher in the tumor compared with its matched normal sample. In cases where normal tissue was not available, a universal pool of cDNA samples was used instead. In these cases, a gene was considered overexpressed in a tumor sample when the difference of expression levels between a tumor sample and the average of all normal samples from the same tissue type was greater than 2 times the standard deviation of all normal samples (i.e., Tumor - average(all normal samples) > 2 x STDEV(all normal samples) ).

Results are shown in Table 1. Number of pairs of tumor samples and matched normal tissue from the same patient are shown for each tumor type. Percentage of the samples with at least two-fold overexpression for each tumor type is provided. A modulator identified by an assay described herein can be further validated for therapeutic effect by administration to a tumor in which the gene is overexpressed. A decrease in tumor growth confirms therapeutic utility of the modulator. Prior to treating a patient with the modulator, the likelihood that the patient will respond to treatment can be diagnosed by obtaining a tumor sample from the patient, and assaying for expression of the gene targeted by the modulator. The expression data for the gene(s) can also be used as a diagnostic marker for disease progression. The assay can be performed by expression analysis as described above, by antibody directed to the gene target, or by any other available detection method.

**Table 1**

| **Seq ID NO** | 4 | 6 |
|---|---|---|
| **Breast** | 11% | 39% |
| **# of Pairs** | 35 | 36 |
| **Colon** | 8% | 8% |
| **# of Pairs** | 40 | 40 |
| **Head And Neck** | 23% | 31% |
| **# of Pairs** | 13 | 13 |
| **Kidney** | 10% | 5% |
| **# of Pairs** | 21 | 21 |
| **Liver** | 22% | 22% |
| **# of Pairs** | 9 | 9 |
| **Lung** | 10% | 8% |
| **# of Pairs** | 39 | 40 |
| **Lymphoma** | 0% | 0% |
| **# of Pairs** | 4 | 4 |
| **Ovary** | 42% | 63% |
| **# of Pairs** | 19 | 19 |
| **Pancreas** | 67% | 67% |
| **# of Pairs** | 12 | 12 |
| **Prostate** | 8% | 17% |
| **# of Pairs** | 24 | 24 |
| **Skin** | 0% | 0% |
| **# of Pairs** | 7 | 7 |
| **Stomach** | 0% | 27% |
| **# of Pairs** | 11 | 11 |
| **Testis** | 0% | 12% |
| **# of Pairs** | 8 | 8 |
| **Thyroid Gland** | 7% | 0% |
| **# of Pairs** | 14 | 14 |
| **Uterus** | 9% | 57% |
| **# of Pairs** | 23 | 23 |

### VII. PRKC functional assays

RNAi experiments were carried out to knock down expression of PRKC (SEQ ID NO:4) in various cell lines using small interfering RNAs (siRNA, Elbashir et al, *supra).* Four siRNAs were used, all of which reduced PRKC mRNA and protein levels.

Effect of PRKC RNAi on cell proliferation and growth. BrdU assay, as described above, was employed to study the effects of decreased PRKC expression on cell proliferation. The results of these experiments indicated that RNAi of PRKC decreases proliferation in SW480 colon cancer and PC3 prostate cancer cells. [³H]-thymidine incorporation assay, as described above, was also employed to study the effects of decreased PRKC expression on cell growth. Results indicated that RNAi of PRKCI decreases proliferation in LOVO and HT20 colon cancer cells, and also in PC3 prostate cancer cells. Standard colony growth assays, as described above, were employed to study the effects of decreased PRKC expression on cell growth. Results indicated that RNAi of PRKC decreases proliferation in SW480 cells.

Effect of PRKC RNAi on apoptosis. Nucleosome ELISA apoptosis assay, as described above, was employed to study the effects of decreased PRKC expression on apoptosis. The results indicated that RNAi of PRKC increased apoptosis in HCT116 colon cancer cells. Apoptosis phospho H2B assay, as described above, was also employed to study the effects of decreased PRKC expression on apoptosis. Results indicated that RNAi of PRKC increases apoptosis in SW480 and PC3 cells. RNAi of PRKC also decreased cell count in SW480 cells. In another apoptosis assay, effects of reduced PRKC expression on DNA fragmentation, PARP activity, caspase activity, and nuclear swelling and condensation were simultaneously measured using the Cellomics™ ArrayScan^{®} HCS System, as described above. Results indicated that RNAi of PRKC has significant effects on caspase activity and nuclear swelling in A549 lung cancer and PC3 prostate cancer cells.

PRKC overexpression analysis. PRKC was overexpressed and tested in colony growth assays as described above. Overexpressed PRKC, in combination with Ras, caused increased growth and formation of foci in NIH3T3 cells. Overexpressed PRKC alone caused increased growth in MDCK cells.

Transcriptional reporter assays. Effects of overexpressed PRKC on expression of various transcription factors was studied. In this assay, rat intestinal epithelial cells (RIEs) cells were co-transfected with reporter constructs containing various transcription factors and luciferase along with PRKC. Luciferase intensity was then measured as the readout for transcriptional activation due to overexpression of the PRKC. Overexpressed PRKC caused an increased expression of SRE (Serum response element) in RIE cells. High Throughput active nuclear beta catenin measurement assay. Beta catenin is a cytoplasmic gene, which when activated, moves into the nucleus. This assay was designed to measure the amount of active beta catenin protein in the nucleus using an anti active beta catenin antibody and a nuclear staining dye. Using this assay, we looked for genes that when knocked out, decrease beta catenin activity, and hence, the amount of active beta catenin in the nucleus. This assay was performed using Cellomics Inc. instrumentation. For this assay, cells were transfected in quadruplicate with siRNAs in 96 well format and stained 72 hours post transfection. The amount of nuclear beta catenin was measured using two different methods. RNAi of PRKC caused a decrease in nuclear beta catenin in SW480 cells.

TOPFLASH beta-catenin reporter assay. Factors of the TCF/LEF HMG domain family (TCFs) exist in vertebrates, Drosophila melanogaster and Caenorhabditis elegans. Upon Wingless/Wnt signaling, Armadillo/beta-catenin associate with nuclear TCFs and contribute a trans-activation domain to the resulting bipartite transcription factor. So, transcriptional activation of TCF target genes by beta-catenin appears to be a central event in development and cellular transformation. Topflash beta-catenin luciferase gene reporter assay is used as a tool to measures activity of various genes in the beta-catenin pathway by transcriptional activation of TCFs (Korinek, V, et al. (1998) Molecular and Cellular Biology 18: 1248-1256). Briefly, cells are co-transfected with TOPFLASH plasmids containing TCF binding sites driving luciferase, and gene of interest. Transfected cells are then analyzed for luciferase activity. RNAi of PRKC caused decreased luciferase activity as compared with normal controls in SW40 and LOVO colon cancer cells, and in LX1 lung cancer cells.

## Claims

1. An in vitro method of identifying a candidate beta catenin pathway modulating agent, said method comprising the steps of:
(a) providing an assay system capable of detecting Protein Kinase C (PRKC) expression and/or kinase activity, said assay system comprising a PRKC nucleic acid having a nucleotide sequence shown as Sequence ID Nos: 1, 2, 4 or 5 or a PRKC polypeptide, wherein said PRKC polypeptide is a PRKC protein encoded by said PRKC nucleic acid, or a fragment or derivative of said PRKC protein,
(b) contacting the assay system with a test agent; and
(c) determining (i) the expression of said PRKC polypeptide or PRKC nucleic acid or (ii) the kinase activity of said PRKC polypeptide in the assay system, wherein a change in said expression or kinase activity between the presence and absence of said test agent identifies the test agent as a candidate modulator of beta catenin expression or activity.

2. The method of Claim 1 wherein the assay system comprises cultured cells that express the PRKC polypeptide.

3. The method of Claim 2 wherein the cultured cells additionally have defective beta catenin function.

4. The method of Claim 1 wherein the assay system includes a screening assay comprising said PRKC polypeptide, and the candidate test agent is a small molecule modulator.

5. The method of Claim 4 wherein the assay is a kinase assay.

6. The method of Claim 1 wherein the assay system includes a binding assay comprising a PRKC polypeptide and the candidate test agent is an antibody.

7. The method of Claim 1 wherein the assay system includes an expression assay comprising a PRKC nucleic acid and the candidate test agent is a nucleic acid modulator.

8. The method of claim 7 wherein the nucleic acid modulator is an antisense oligomer.

9. The method of Claim 7 wherein the nucleic acid modulator is a phosphothioate morpholino oligomer (PMO).

10. The method of Claim 1 additionally comprising:
(d) administering the test agent to an in vitro model system comprising cells defective in beta catenin function and, detecting a phenotypic change in the model system that indicates that the beta catenin function is restored.

11. An in vitro method for modulating a beta catenin pathway in a cell comprising contacting the cell with an agent that specifically modulates
(i) the expression of PRKC nucleic acid or polypeptide and/or
(ii) PRKC polypeptide kinase activity;
wherein said PRKC nucleic acid has a nucleotide sequence shown as Sequence ID Nos: 1, 2, 4 or 5;
wherein said PRKC polypeptide is a PRKC protein encoded by said PRKC nucleic acid, or a fragment or derivative of said PRKC protein; and
wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

12. An agent that specifically modulates
(i) the expression of PRKC nucleic acid or polypeptide and/or
(ii) PRKC polypeptide kinase activity
for use in the treatment of a disease or disorder selected from an angiogenic, apoptotic and cell proliferation disease or disorder;
wherein said PRKC nucleic acid has a nucleotide sequence shown as Sequence ID Nos: 1, 2, 4 or 5;
wherein said PRKC polypeptide is a PRKC protein encoded by said PRKC nucleic acid, or a fragment or derivative of said PRKC protein; and
wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

13. Use of an agent that specifically modulates (i) expression of PRKC nucleic acid or polypeptide and/or (ii) PRKC polypeptide kinase activity
in the preparation of a medicament for the treatment of a disease or disorder selected from an angiogenic, apoptotic and cell proliferation disease or disorder;
wherein said PRKC nucleic acid has a nucleotide sequence shown as Sequence ID Nos: 1, 2, 4 or 5;
wherein said PRKC polypeptide is a PRKC protein encoded by said PRKC nucleic acid, or a fragment or derivative of said PRKC protein; and
wherein said agent is a PRKC specific antibody, a PRKC specific T cell antigen receptor, a PRKC specific antisense oligomer, a PRKC specific double-stranded RNA, a PRKC specific phosphothioate morpholino oligomer, or said PRKC protein.

14. The agent according to claim 12 or the use according to claim 13, wherein the agent is a PRKC specific antibody.

15. The method of Claim 1, comprising the additional steps of:
(d) providing an in vitro secondary assay system capable of detecting a change in the beta catenin pathway comprising cultured cells expressing said PRKC,
(e) contacting the secondary assay system with the test agent of (b) or an agent derived therefrom; and
(f) determining a change in the beta catenin pathway in the secondary assay system, wherein a change in the beta catenin pathway between the presence and absence of said test agent or agent derived therefrom confirms the candidate test agent as a beta catenin modulating agent.

16. The method of Claim 15 wherein the cultured cells mis-express a beta catenin pathway gene.

17. An in vitro method for diagnosing a disorder or disease associated with a defect in the beta catenin pathway in a biological sample from a patient comprising:
(a) contacting the sample with a probe for PRKC nucleic acid or polypeptide expression, wherein said PRKC nucleic acid has a nucleotide sequence shown as Sequence ID Nos: 1, 2, 4 or 5 and wherein said PRKC polypeptide is a PRKC protein encoded by said PRKC nucleic acid, or a fragment or derivative of said PRKC protein;
(b) comparing results from step (a) with a control; and
(c) determining whether step (b) indicates a likelihood of a disorder or disease associated with a defect in the beta catenin pathway, wherein the disease or disorder is selected from an angiogenic, apoptotic and cell proliferation disease or disorder.

18. The method of claim 17, wherein said disease is cancer.

19. The method according to claim 18, wherein said cancer is a cancer as shown in Table 1 as having >25% expression level.

20. The method according to claim 11, wherein the cell is a cell defective in beta catenin function and the agent is a PRKC specific antibody or a PRKC specific T cell antigen receptor, whereby beta catenin function is restored.

21. The agent according to claim 12 or the use according to claim 13, wherein the agent is a PRKC specific antibody or a PRKC specific T cell antigen receptor or a PRKC protein, whereby beta catenin function is restored.

22. The agent or the use according to claim 21, wherein the treatment is of a vertebrate animal predetermined to have a disease or disorder resulting from a defect in beta catenin function.

23. The method according to claim 20 or the agent or use according to claim 22 wherein the candidate modulator is a PRKC specific antibody.

24. The method according to claim 11, wherein said cell is a mammalian cell.

25. The agent according to claim 12 or the use according to claim 13, wherein said treatment is treatment of a mammal.

26. The agent or use according to claim 25, wherein the mammal is predetermined to have a pathology associated with a defect in the beta catenin pathway.

## Patentansprüche

1. Ein In-vitro-Verfahren zum Identifizieren eines den Beta-Catenin-Weg modulierenden Wirkstoffkandidaten, wobei das Verfahren die folgenden Schritte beinhaltet:
(a) Bereitstellen eines Assay-Systems, das in der Lage ist, die Expression und/oder die Kinaseaktivität von Proteinkinase C (PRKC) zu erkennen, wobei das Assay-System eine PRKC-Nukleinsäure mit einer als SEQ ID NO: 1, 2, 4 oder 5 gezeigten Nukleotidsequenz oder ein PRKC-Polypeptid beinhaltet, wobei das PRKC-Polypeptid ein von der PRKC-Nukleinsäure kodiertes PRKC-Protein oder ein Fragment oder Derivat des PRKC-Proteins ist,
(b) In-Kontakt-Bringen des Assay-Systems mit einem Testwirkstoff und
(c) Bestimmen (i) der Expression des PRKC-Polypeptids oder der PRKC-Nukleinsäure oder (ii) der Kinaseaktivität des PRKC-Polypeptids in dem Assay-System, wobei eine Änderung der Expression oder Kinaseaktivität zwischen dem Vorhandensein und der Abwesenheit des Testwirkstoffs den Testwirkstoff als einen Modulatorkandidaten für Beta-Catenin-Expression oder -Aktivität identifiziert.

2. Verfahren gemäß Anspruch 1, wobei das Assay-System kultivierte Zellen beinhaltet, die das PRKC-Polypeptid exprimieren.

3. Verfahren gemäß Anspruch 2, wobei die kultivierten Zellen zusätzlich eine fehlerhafte Beta-Catenin-Funktion aufweisen.

4. Verfahren gemäß Anspruch 1, wobei das Assay-System einen Screening-Assay umfasst, der das PRKC-Polypeptid beinhaltet, und der Testwirkstoffkandidat ein Modulator aus einem kleinen Molekül ist.

5. Verfahren gemäß Anspruch 4, wobei der Assay ein Kinase-Assay ist.

6. Verfahren gemäß Anspruch 1, wobei das Assay-System einen Bindungsassay umfasst, der ein PRKC-Polypeptid beinhaltet, und der Testwirkstoffkandidat ein Antikörper ist.

7. Verfahren gemäß Anspruch 1, wobei das Assay-System einen Expressionsassay umfasst, der eine PRKC-Nukleinsäure beinhaltet, und der Testwirkstoffkandidat ein Modulator aus Nukleinsäure ist.

8. Verfahren gemäß Anspruch 7, wobei der Modulator aus Nukleinsäure ein Antisense-Oligomer ist.

9. Verfahren gemäß Anspruch 7, wobei der Modulator aus Nukleinsäure ein Phosphothioatmorpholino-Oligomer (PMO) ist.

10. Verfahren gemäß Anspruch 1, das zusätzlich Folgendes beinhaltet:
(d) Verabreichen des Testwirkstoffs an ein In-vitro-Modellsystem, das hinsichtlich der Beta-Catenin-Funktion fehlerhafte Zellen beinhaltet, und Erkennen einer phänotypischen Änderung in dem Modellsystem, die anzeigt, dass die Beta-Catenin-Funktion wiederhergestellt ist.

11. Ein In-vitro-Verfahren zum Modulieren eines Beta-Catenin-Wegs in einer Zelle, das das In-Kontakt-Bringen der Zelle mit einem Wirkstoff beinhaltet, der spezifisch Folgendes moduliert:
(i) die Expression von PRKC-Nukleinsäure oder -Polypeptid und/oder
(ii) die PRKC-Polypeptid-Kinaseaktivität;
wobei die PRKC-Nukleinsäure eine als SEQ ID NO: 1, 2, 4 oder 5 gezeigte Nukleotidsequenz aufweist;
wobei das PRKC-Polypeptid ein von der PRKC-Nukleinsäure kodiertes PRKC-Protein oder ein Fragment oder Derivat des PRKC-Proteins ist und
wobei der Wirkstoff ein PRKC-spezifischer Antikörper, ein PRKC-spezifischer T-Zell-Antigenrezeptor, ein PRKC-spezifisches Antisense-Oligomer, eine PRKC-spezifische doppelsträngige RNA, ein PRKC-spezifisches Phosphothioatmorpholino-Oligomer oder das PRKC-Protein ist.

12. Ein Wirkstoff, der spezifisch Folgendes moduliert:
(i) die Expression von PRKC-Nukleinsäure oder -Polypeptid und/oder
(ii) die PRKC-Polypeptid-Kinaseaktivität zur Verwendung bei der Behandlung einer Krankheit oder Störung, ausgewählt aus einer die Angiogenese, Apoptose oder Zellproliferation betreffenden Krankheit oder Störung;
wobei die PRKC-Nukleinsäure eine als SEQ ID NO: 1, 2, 4 oder 5 gezeigte Nukleotidsequenz aufweist;
wobei das PRKC-Polypeptid ein von der PRKC-Nukleinsäure kodiertes PRKC-Protein oder ein Fragment oder Derivat des PRKC-Proteins ist und
wobei der Wirkstoff ein PRKC-spezifischer Antikörper, ein PRKC-spezifischer T-Zell-Antigenrezeptor, ein PRKC-spezifisches Antisense-Oligomer, eine PRKC-spezifische doppelsträngige RNA, ein PRKC-spezifisches Phosphothioatmorpholino-Oligomer oder das PRKC-Protein ist.

13. Eine Verwendung eines Wirkstoffs, der spezifisch (i) die Expression von PRKC-Nukleinsäure oder -Polypeptid und/oder (ii) die PRKC-Polypeptid-Kinaseaktivität moduliert,
bei der Zubereitung eines Medikaments für die Behandlung einer Krankheit oder Störung, ausgewählt aus einer die Angiogenese, Apoptose oder Zellproliferation betreffenden Krankheit oder Störung;
wobei die PRKC-Nukleinsäure eine als SEQ ID NO: 1, 2, 4 oder 5 gezeigte Nukleotidsequenz aufweist;
wobei das PRKC-Polypeptid ein von der PRKC-Nukleinsäure kodiertes PRKC-Protein oder ein Fragment oder Derivat des PRKC-Proteins ist und
wobei der Wirkstoff ein PRKC-spezifischer Antikörper, ein PRKC-spezifischer T-Zell-Antigenrezeptor, ein PRKC-spezifisches Antisense-Oligomer, eine PRKC-spezifische doppelsträngige RNA, ein PRKC-spezifisches Phosphothioatmorpholino-Oligomer oder das PRKC-Protein ist.

14. Wirkstoff gemäß Anspruch 12 oder Verwendung gemäß Anspruch 13, wobei der Wirkstoff ein PRKC-spezifischer Antikörper ist.

15. Verfahren gemäß Anspruch 1, das die folgenden zusätzlichen Schritte beinhaltet:
(d) Bereitstellen eines sekundären In-vitro-Assay-Systems, das in der Lage ist, eine Änderung des Beta-Catenin-Wegs zu erkennen, das das PRKC exprimierende kultivierte Zellen beinhaltet,
(e) In-Kontakt-Bringen des sekundären Assay-Systems mit dem Testwirkstoff aus (b) oder einem davon abgeleiteten Wirkstoff und
(f) Bestimmen einer Änderung des Beta-Catenin-Wegs in dem sekundären Assay-System, wobei eine Änderung des Beta-Catenin-Wegs zwischen dem Vorhandensein und der Abwesenheit des Testwirkstoffs oder des davon abgeleiteten Wirkstoffs den Testwirkstoffkandidaten als Beta-Catenin modulierenden Wirkstoff bestätigt.

16. Verfahren gemäß Anspruch 15, wobei die kultivierten Zellen ein Gen des Beta-Catenin-Wegs falsch exprimieren.

17. Ein In-vitro-Verfahren zum Diagnostizieren einer Störung oder Krankheit, die mit einem Defekt in dem Beta-Catenin-Weg verbunden ist, in einer biologischen Probe von einem Patienten, das Folgendes beinhaltet:
(a) In-Kontakt-Bringen der Probe mit einer Sonde für PRKC-Nukleinsäure- oder -Polypeptidexpression, wobei die PRKC-Nukleinsäure eine als SEQ ID NO: 1, 2, 4 oder 5 gezeigte Nukleinsequenz aufweist und wobei das PRKC-Polypeptid ein von der PRKC-Nukleinsäure kodiertes PRKC-Protein oder ein Fragment oder Derivat des PRKC-Proteins ist;
(b) Vergleichen der Ergebnisse aus Schritt (a) mit einer Kontrolle und
(c) Bestimmen, ob Schritt (b) eine Wahrscheinlichkeit einer mit einem Defekt in dem Beta-Catenin-Weg verbundenen Störung oder Krankheit anzeigt, wobei die Krankheit oder Störung aus einer die Angiogenese, Apoptose oder Zellproliferation betreffenden Krankheit oder Störung ausgewählt ist.

18. Verfahren gemäß Anspruch 17, wobei die Krankheit Krebs ist.

19. Verfahren gemäß Anspruch 18, wobei der Krebs ein wie in Tabelle 1 mit einem Expressionsgrad von >25 % gezeigter Krebs ist.

20. Verfahren gemäß Anspruch 11, wobei die Zelle eine Zelle mit fehlerhafter Beta-Catenin-Funktion ist und der Wirkstoff ein PRKC-spezifischer Antikörper oder ein PRKC-spezifischer T-Zell-Antigenrezeptor ist, wodurch die Beta-Catenin-Funktion wiederhergestellt wird.

21. Wirkstoff gemäß Anspruch 12 oder Verwendung gemäß Anspruch 13, wobei der Wirkstoff ein PRKC-spezifischer Antikörper oder ein PRKC-spezifischer T-Zell-Antigenrezeptor oder ein PRKC-Protein ist, wodurch die Beta-Catenin-Funktion wiederhergestellt wird.

22. Wirkstoff oder Verwendung gemäß Anspruch 21, wobei die Behandlung die eines Wirbeltiers, von dem zuvor bestimmt wurde, dass es eine aus einem Defekt bei der Beta-Catenin-Funktion resultierende Krankheit oder Störung aufweist, ist.

23. Verfahren gemäß Anspruch 20 oder Wirkstoff oder Verwendung gemäß Anspruch 22, wobei der Modulatorkandidat ein PRKC-spezifischer Antikörper ist.

24. Verfahren gemäß Anspruch 11, wobei die Zelle eine Säugetierzelle ist.

25. Wirkstoff gemäß Anspruch 12 oder Verwendung gemäß Anspruch 13, wobei die Behandlung eine Behandlung eines Säugetieres ist.

26. Wirkstoff oder Verwendung gemäß Anspruch 25, wobei zuvor bestimmt wurde, dass das Säugetier einen mit einem Defekt im Beta-Catenin-Weg verbundenen pathologischen Befund aufweist.

## Revendications

1. Une méthode in vitro pour identifier un agent de modulation de voie bêta-caténine candidat, ladite méthode comprenant les étapes de :
(a) fournir un système d'épreuve capable de détecter une expression de protéine kinase C (PRKC) et / ou une activité kinase, ledit système d'épreuve comprenant un acide nucléique PRKC ayant une séquence de nucléotides montrée comme étant les Séquences ID N° : 1, 2, 4 ou 5 ou un polypeptide PRKC, où ledit polypeptide PRKC est une protéine PRKC codée par ledit acide nucléique PRKC, ou un fragment ou un dérivé de ladite protéine PRKC,
(b) mettre en contact le système d'épreuve avec un agent de test ; et
(c) déterminer (i) l'expression dudit polypeptide PRKC ou de l'acide nucléique PRKC ou (ii) l'activité kinase dudit polypeptide PRKC dans le système d'épreuve, où un changement dans ladite expression ou ladite activité kinase entre la présence et l'absence dudit agent de test identifie l'agent de test comme étant un modulateur candidat de l'expression ou de l'activité de la bêta-caténine.

2. La méthode de la revendication 1 où le système d'épreuve comprend des cellules cultivées qui expriment le polypeptide PRKC.

3. La méthode de la revendication 2 où les cellules cultivées ont de plus une fonction bêta-caténine défectueuse.

4. La méthode de la revendication 1 où le système d'épreuve comporte une épreuve de criblage comprenant ledit polypeptide PRKC, et l'agent de test candidat est une petite molécule modulateur.

5. La méthode de la revendication 4 où l'épreuve est une épreuve kinase.

6. La méthode de la revendication 1 où le système d'épreuve comporte une épreuve de liaison comprenant un polypeptide PRKC, et l'agent de test candidat est un anticorps.

7. La méthode de la revendication 1 où le système d'épreuve comporte une épreuve d'expression comprenant un acide nucléique PRKC, et l'agent de test candidat est un acide nucléique modulateur.

8. La méthode de la revendication 7 où l'acide nucléique modulateur est un oligomère antisens.

9. La méthode de la revendication 7 où l'acide nucléique modulateur est un oligomère morpholino phosphothioate (PMO).

10. La méthode de la revendication 1 comprenant de plus :
(d) administrer l'agent de test à un système modèle in vitro comprenant des cellules défectueuses en terme de fonction bêta-caténine, et détecter un changement phénotypique dans le système modèle qui indique que la fonction bêta-caténine est restaurée.

11. Une méthode in vitro pour moduler une voie bêta-caténine dans une cellule comprenant mettre en contact la cellule avec un agent qui module de façon spécifique
(i) l'expression de l'acide nucléique ou du polypeptide PRKC et / ou
(ii) l'activité kinase du polypeptide PRKC ;
où ledit acide nucléique PRKC a une séquence de nucléotides montrée comme étant les Séquences ID N° : 1, 2, 4 ou 5 ;
où ledit polypeptide PRKC est une protéine PRKC codée par ledit acide nucléique PRKC, ou un fragment ou un dérivé de ladite protéine PRKC ; et
où ledit agent est un anticorps spécifique de PRKC, un récepteur antigénique des cellules T spécifique de PRKC, un oligomère antisens spécifique de PRKC, un ARN double brin spécifique de PRKC, un oligomère morpholino phosphothioate spécifique de PRKC, ou ladite protéine PRKC.

12. Un agent qui module de façon spécifique
(i) l'expression de l'acide nucléique ou du polypeptide PRKC et / ou
(ii) l'activité kinase du polypeptide PRKC pour l'utiliser dans le traitement d'une maladie ou d'un trouble sélectionnés parmi une maladie ou un trouble angiogénique, apoptotique et à prolifération cellulaire ;
où ledit acide nucléique PRKC a une séquence de nucléotides montrée comme étant les Séquences ID N° : 1, 2, 4 ou 5 ;
où ledit polypeptide PRKC est une protéine PRKC codée par ledit acide nucléique PRKC, ou un fragment ou un dérivé de ladite protéine PRKC ; et
où ledit agent est un anticorps spécifique de PRKC, un récepteur antigénique des cellules T spécifique de PRKC, un oligomère antisens spécifique de PRKC, un ARN double brin spécifique de PRKC, un oligomère morpholino phosphothioate spécifique de PRKC, ou ladite protéine PRKC.

13. Utilisation d'un agent qui module de façon spécifique (i) l'expression de l'acide nucléique ou du polypeptide PRKC et / ou (ii) l'activité kinase du polypeptide PRKC
dans la préparation d'un médicament pour le traitement d'une maladie ou d'un trouble sélectionnés parmi une maladie ou un trouble angiogénique, apoptotique et à prolifération cellulaire ;
où ledit acide nucléique PRKC a une séquence de nucléotides montrée comme étant les Séquences ID N° : 1, 2, 4 ou 5 ;
où ledit polypeptide PRKC est une protéine PRKC codée par ledit acide nucléique PRKC, ou un fragment ou un dérivé de ladite protéine PRKC ; et
où ledit agent est un anticorps spécifique de PRKC, un récepteur antigénique des cellules T spécifique de PRKC, un oligomère antisens spécifique de PRKC, un ARN double brin spécifique de PRKC, un oligomère morpholino phosphothioate spécifique de PRKC, ou ladite protéine PRKC.

14. L'agent selon la revendication 12 ou l'utilisation selon la revendication 13, où l'agent est un anticorps spécifique de PRKC.

15. La méthode de la revendication 1, comprenant les étapes supplémentaires de :
(d) fournir un système d'épreuve in vitro secondaire capable de détecter un changement dans la voie bêta-caténine comprenant des cellules cultivées exprimant ledit PRKC,
(e) mettre en contact le système d'épreuve secondaire avec l'agent de test de (b) ou un agent dérivé de celui-ci ; et
(f) déterminer un changement dans la voie bêta-caténine dans le système d'épreuve secondaire, où un changement dans la voie bêta-caténine entre la présence et l'absence dudit agent de test ou de l'agent dérivé de celui-ci confirme que l'agent de test candidat est un agent modulant la bêta-caténine.

16. La méthode de la revendication 15 où les cellules cultivées expriment de façon modifiée un gène de voie bêta-caténine.

17. Une méthode in vitro pour diagnostiquer un trouble ou une maladie associés à un défaut dans la voie bêta-caténine dans un échantillon biologique provenant d'un patient comprenant :
(a) mettre en contact l'échantillon avec une sonde pour détecter l'expression de l'acide nucléique ou du polypeptide PRKC, où ledit acide nucléique PRKC a une séquence de nucléotides montrée comme étant les Séquences ID N° : 1, 2, 4 ou 5 et où ledit polypeptide PRKC est une protéine PRKC codée par ledit acide nucléique PRKC, ou un fragment ou un dérivé de ladite protéine PRKC ;
(b) comparer les résultats de l'étape (a) à un témoin ; et
(c) déterminer si l'étape (b) indique un risque d'un trouble
ou d'une maladie associés à un défaut dans la voie bêta-caténine, où la maladie ou le trouble sont sélectionnés parmi une maladie ou un trouble angiogénique, apoptotique et à prolifération cellulaire.

18. La méthode de la revendication 17, où ladite maladie est un cancer.

19. La méthode selon la revendication 18, où ledit cancer est un cancer tel que montré sur le Tableau 1 ayant un niveau d'expression > 25 %.

20. La méthode selon la revendication 11, où la cellule est une cellule défectueuse en termes de fonction bêta-caténine et l'agent est un anticorps spécifique de PRKC ou un récepteur antigénique des cellules T spécifique de PRKC, grâce à quoi la fonction bêta-caténine est restaurée.

21. L'agent selon la revendication 12 ou l'utilisation selon la revendication 13, où l'agent est un anticorps spécifique de PRKC ou un récepteur antigénique des cellules T spécifique de PRKC ou une protéine PRKC, grâce à quoi la fonction béta-caténine est restaurée.

22. L'agent ou l'utilisation selon la revendication 21, où le traitement est celui d'un animal vertébré chez lequel ont été décelés une maladie ou un trouble résultant d'un défaut dans la fonction bêta-caténine.

23. La méthode selon la revendication 20 ou l'agent ou l'utilisation selon la revendication 22, où le modulateur candidat est un anticorps spécifique de PRKC.

24. La méthode selon la revendication 11, où ladite cellule est une cellule mammalienne.

25. L'agent selon la revendication 12 ou l'utilisation selon la revendication 13, où ledit traitement est le traitement d'un mammifère.

26. L'agent ou l'utilisation selon la revendication 25, où il a été décelé chez le mammifère une pathologie associée à un défaut dans la voie bêta-caténine.
